# EUROPEAN PATENT APPLICATION

(11) **EP 2 505 640 A1**
(43) Date of publication of application: **03.10.2012**
(21) Application number: 11382084.9
(22) Date of filing: 29.03.2011
(51) Int. Cl.: C12N 7/04, C07K 14/08, C12N 15/62

(54) **Vaccine compositions for birnavirus-borne diseases**

(71) Applicant: Neo Virnatech, S.L., 08110 Barcelona (ES)
(72) Inventor: Oña Blanco, Ana María, E-28037, Madrid (ES)
(74) Representative: ABG Patentes, S.L.

(57) **Abstract**

The invention relates to birnavirus immunogens derived from the VP2 polypeptide, wherein said immunogens are formed by the projection domain of said polypeptide lacking the AA' hairpin region. The invention also relates to conjugates comprising said immunogen and a co-stimulatory molecule. The invention further relates to vaccine compositions comprising the above immunogens as well as to the uses thereof for the treatment of birnavirus-borne infections.

SEQUENCE LISTING

<110>  Neo VirnaTech S.L.

<120>  VACCINE COMPOSITIONS FOR BIRNAVIRUS-BORNE DISEASES

<130>  P6305EP00

<160>  15

<170>  PatentIn version 3.5

<210>  1
<211>  141
<212>  PRT
<213>  Artificial

<220>
<223>  IBDV VP2 P domain region

<400>  1

Ser Asp Arg Pro Arg Val Tyr Thr Ile Thr Ala Ala Asp Asp Tyr Gln
1               5                   10                  15

Phe Ser Ser Gln Tyr Gln Pro Gly Gly Val Thr Ile Thr Leu Phe Ser
            20                  25                  30

Ala Asn Ile Asp Ala Ile Thr Ser Leu Ser Val Gly Gly Glu Leu Val
        35                  40                  45

Phe Arg Thr Ser Val His Gly Leu Val Leu Gly Ala Thr Ile Tyr Leu
    50                  55                  60

Ile Gly Phe Asp Gly Thr Thr Val Ile Thr Arg Ala Val Ala Ala Asn
65                  70                  75                  80

Asn Gly Leu Thr Thr Gly Thr Asp Asn Leu Met Pro Phe Asn Leu Val
                85                  90                  95

Ile Pro Thr Asn Glu Ile Thr Gln Pro Ile Thr Ser Ile Lys Leu Glu
            100                 105                 110

Ile Val Thr Ser Lys Ser Gly Gly Gln Ala Gly Asp Gln Met Ser Trp
        115                 120                 125

Ser Ala Arg Gly Ser Leu Ala Val Thr Ile His Gly Gly
    130                 135                 140

<210>  2
<211>  139
<212>  PRT
<213>  Artificial

<220>
<223>  IBDV VP2 P domain region

<400>  2

Ser Asp Arg Pro Arg Val Tyr Thr Ile Thr Ala Ala Asp Asp Tyr Gln
1               5                   10                  15

Phe Ser Ser Gln Tyr Gln Pro Gly Gly Val Thr Ile Thr Leu Phe Ser
            20                  25                  30

Ala Asn Ile Asp Ala Ile Thr Ser Leu Ser Val Gly Gly Glu Leu Val
        35                  40                  45

Phe Arg Thr Ser Val His Gly Leu Val Leu Gly Ala Thr Ile Tyr Leu
    50                  55                  60

Ile Gly Phe Asp Gly Thr Thr Val Ile Thr Arg Ala Val Ala Ala Asn
65                  70                  75                  80

Asn Gly Leu Thr Thr Gly Thr Asp Asn Leu Met Pro Phe Asn Leu Val
                85                  90                  95

Ile Pro Thr Asn Glu Ile Thr Gln Pro Ile Thr Ser Ile Lys Leu Glu
            100                 105                 110

Ile Val Thr Ser Lys Ser Gly Gly Gln Ala Gly Asp Gln Met Ser Trp
        115                 120                 125

Ser Ala Arg Gly Ser Leu Ala Val Thr Ile His
    130                 135

<210>  3
<211>  441
<212>  PRT
<213>  Infectious Bursal disease virus

<400>  3

Met Thr Asn Leu Gln Asp Gln Thr Gln Gln Ile Val Pro Phe Ile Arg
1               5                   10                  15

Ser Leu Leu Met Pro Thr Thr Gly Pro Ala Ser Ile Pro Asp Asp Thr
            20                  25                  30

Leu Glu Lys His Thr Leu Arg Ser Glu Thr Ser Thr Tyr Asn Leu Thr
        35                  40                  45

Val Gly Asp Thr Gly Ser Gly Leu Ile Val Phe Phe Pro Gly Phe Pro
    50                  55                  60

Gly Ser Ile Val Gly Ala His Tyr Thr Leu Gln Ser Asn Gly Asn Tyr
65                  70                  75                  80

Lys Phe Asp Gln Met Leu Leu Thr Ala Gln Asn Leu Pro Ala Ser Tyr
                85                  90                  95

Asn Tyr Cys Arg Leu Val Ser Arg Ser Leu Thr Val Arg Ser Ser Thr
            100                 105                 110

Leu Pro Gly Gly Val Tyr Ala Leu Asn Gly Thr Ile Asn Ala Val Thr
        115                 120                 125

Phe Gln Gly Ser Leu Ser Glu Leu Thr Asp Val Ser Tyr Asn Gly Leu
    130                 135                 140

Met Ser Ala Thr Ala Asn Ile Asn Asp Lys Ile Gly Asn Val Leu Val
145                 150                 155                 160

Gly Glu Gly Val Thr Val Leu Ser Leu Pro Thr Ser Tyr Asp Leu Gly
                165                 170                 175

Tyr Val Arg Leu Gly Asp Pro Ile Pro Ala Ile Gly Leu Asp Pro Lys
            180                 185                 190

Met Val Ala Thr Cys Asp Ser Ser Asp Arg Pro Arg Val Tyr Thr Ile
        195                 200                 205

Thr Ala Ala Asp Asp Tyr Gln Phe Ser Ser Gln Tyr Gln Pro Gly Gly
    210                 215                 220

Val Thr Ile Thr Leu Phe Ser Ala Asn Ile Asp Ala Ile Thr Ser Leu
225                 230                 235                 240

Ser Val Gly Gly Glu Leu Val Phe Gln Thr Ser Val His Gly Leu Val
                245                 250                 255

Leu Gly Ala Thr Ile Tyr Leu Ile Gly Phe Asp Gly Thr Thr Val Ile
            260                 265                 270

Thr Arg Ala Val Ala Ala Asn Asn Gly Leu Thr Thr Gly Thr Asp Asn
        275                 280                 285

Leu Met Pro Phe Asn Leu Val Ile Ser Thr Asn Glu Ile Thr Gln Pro
    290                 295                 300

Ile Thr Ser Ile Lys Leu Glu Ile Val Thr Ser Lys Ser Gly Gly Gln
305                 310                 315                 320

Ala Gly Asp Gln Met Ser Trp Ser Ala Lys Gly Ser Leu Ala Val Thr
                325                 330                 335

Ile His Gly Gly Asn Tyr Pro Gly Ala Leu Arg Pro Val Thr Leu Val
            340                 345                 350

Ala Tyr Glu Arg Val Ala Thr Gly Ser Val Val Thr Val Ala Gly Val
        355                 360                 365

Ser Asn Phe Glu Leu Ile Pro Asn Pro Glu Leu Ala Lys Asn Leu Val
    370                 375                 380

Thr Glu Tyr Gly Arg Phe Asp Pro Gly Ala Met Asn Tyr Thr Lys Leu
385                 390                 395                 400

Ile Leu Ser Glu Arg Asp Arg Leu Gly Ile Lys Thr Val Trp Pro Thr
                405                 410                 415

Arg Glu Tyr Thr Asp Phe Arg Glu Tyr Phe Met Glu Val Ala Asp Leu
            420                 425                 430

Asn Ser Pro Leu Lys Ile Ala Gly Ala
        435                 440

<210>  4
<211>  442
<212>  PRT
<213>  Infectious Pancreatic necrosis virus

<400>  4

Met Asn Thr Asn Lys Ala Thr Ala Thr Tyr Leu Lys Ser Ile Met Leu
1               5                   10                  15

Pro Glu Thr Gly Pro Ala Ser Ile Pro Asp Asp Ile Thr Glu Arg His
            20                  25                  30

Ile Leu Lys Gln Glu Thr Ser Ser Tyr Asn Leu Glu Val Ser Glu Ser
        35                  40                  45

Gly Ser Gly Val Leu Val Cys Phe Pro Gly Ala Pro Gly Ser Arg Ile
    50                  55                  60

Gly Ala His Tyr Arg Trp Asn Ala Asn Gln Thr Gly Leu Glu Phe Asp
65                  70                  75                  80

Gln Trp Leu Glu Thr Ser Gln Asp Leu Lys Lys Ala Phe Asn Tyr Gly
                85                  90                  95

Arg Leu Ile Ser Arg Lys Tyr Asp Ile Gln Ser Ser Thr Leu Pro Ala
            100                 105                 110

Gly Leu Tyr Ala Leu Asn Gly Thr Leu Asn Ala Ala Thr Phe Glu Gly
        115                 120                 125

Ser Leu Ser Glu Val Glu Ser Leu Thr Tyr Asn Ser Leu Met Ser Leu
    130                 135                 140

Thr Thr Asn Pro Gln Asp Lys Val Asn Asn Gln Leu Val Thr Lys Gly
145                 150                 155                 160

Val Thr Val Leu Asn Leu Pro Thr Gly Phe Asp Lys Pro Tyr Val Arg
                165                 170                 175

Leu Glu Asp Glu Thr Pro Gln Gly Leu Gln Ser Met Asn Gly Ala Lys
            180                 185                 190

Met Arg Cys Thr Ala Ala Ile Ala Pro Arg Arg Tyr Glu Ile Asp Leu
        195                 200                 205

Pro Ser Gln Arg Leu Pro Pro Val Pro Ala Thr Gly Thr Leu Thr Thr
    210                 215                 220

Leu Tyr Glu Gly Asn Ala Asp Ile Val Asn Ser Thr Thr Val Thr Gly
225                 230                 235                 240

Asp Ile Asn Phe Ser Leu Ala Glu Gln Pro Ala Asp Glu Thr Lys Phe
                245                 250                 255

Asp Phe Gln Leu Asp Phe Met Gly Leu Asp Asn Asp Val Pro Val Val
            260                 265                 270

Thr Val Val Ser Ser Val Leu Ala Thr Asn Asp Asn Tyr Arg Gly Val
        275                 280                 285

Ser Ala Lys Met Thr Gln Ser Ile Pro Thr Glu Asn Ile Thr Lys Pro
    290                 295                 300

Ile Thr Arg Val Lys Leu Ser Tyr Lys Ile Asn Gln Gln Thr Ala Ile
305                 310                 315                 320

Gly Asn Val Ala Thr Leu Gly Thr Met Gly Pro Ala Ser Val Ser Phe
                325                 330                 335

Ser Ser Gly Asn Gly Asn Val Pro Gly Val Leu Arg Pro Ile Thr Leu
            340                 345                 350

Val Ala Tyr Glu Lys Met Thr Pro Leu Ser Ile Leu Thr Val Ala Gly
        355                 360                 365

Val Ser Asn Tyr Glu Leu Ile Pro Asn Pro Glu Leu Leu Lys Asn Met
    370                 375                 380

Val Thr Arg Tyr Gly Lys Tyr Asp Pro Glu Gly Leu Asn Tyr Ala Lys
385                 390                 395                 400

Met Ile Leu Ser His Arg Glu Glu Leu Asp Ile Arg Thr Val Trp Arg
                405                 410                 415

Thr Glu Glu Tyr Lys Glu Arg Thr Arg Val Phe Asn Glu Ile Thr Asp
            420                 425                 430

Phe Ser Ser Asp Leu Pro Thr Ser Lys Ala
        435                 440

<210>  5
<211>  431
<212>  PRT
<213>  Drosophila X virus

<400>  5

Met Asn Thr Thr Asn Glu Tyr Leu Lys Thr Leu Leu Asn Pro Ala Gln
1               5                   10                  15

Phe Ile Ser Asp Ile Pro Asp Asp Ile Met Ile Arg His Val Asn Ser
            20                  25                  30

Ala Gln Thr Ile Thr Tyr Asn Leu Lys Ser Gly Ala Ser Gly Thr Gly
        35                  40                  45

Leu Ile Val Val Tyr Pro Asn Thr Pro Ser Ser Ile Ser Gly Phe His
    50                  55                  60

Tyr Ile Trp Asp Ser Ala Thr Ser Asn Trp Val Phe Asp Gln Tyr Ile
65                  70                  75                  80

Tyr Thr Ala Gln Glu Leu Lys Asp Ser Tyr Asp Tyr Gly Arg Leu Ile
                85                  90                  95

Ser Gly Ser Leu Ser Ile Lys Ser Ser Thr Leu Pro Ala Gly Val Tyr
            100                 105                 110

Ala Leu Asn Gly Thr Phe Asn Ala Val Trp Phe Gln Gly Thr Leu Ser
        115                 120                 125

Glu Val Ser Asp Tyr Ser Tyr Asp Arg Ile Leu Ser Ile Thr Ser Asn
    130                 135                 140

Pro Leu Asp Lys Val Gly Asn Val Leu Val Gly Asp Gly Ile Glu Val
145                 150                 155                 160

Leu Ser Leu Pro Gln Gly Phe Asn Asn Pro Tyr Val Arg Leu Gly Asp
                165                 170                 175

Lys Ser Pro Ser Thr Leu Ser Ser Pro Thr His Ile Thr Asn Thr Ser
            180                 185                 190

Gln Asn Leu Ala Thr Gly Gly Ala Tyr Met Ile Pro Val Thr Thr Val
        195                 200                 205

Pro Gly Gln Gly Phe His Asn Lys Glu Phe Ser Ile Asn Val Asp Ser
    210                 215                 220

Val Gly Pro Val Asp Ile Leu Trp Ser Gly Gln Met Thr Met Gln Asp
225                 230                 235                 240

Glu Trp Thr Val Thr Ala Asn Tyr Gln Pro Leu Asn Ile Ser Gly Thr
                245                 250                 255

Leu Ile Ala Asn Ser Gln Arg Thr Leu Thr Trp Ser Asn Thr Gly Val
            260                 265                 270

Ser Asn Gly Ser His Tyr Met Asn Met Asn Asn Leu Asn Val Ser Leu
        275                 280                 285

Phe His Glu Asn Pro Pro Pro Glu Pro Val Ala Ala Ile Lys Ile Asn
    290                 295                 300

Ile Asn Tyr Gly Asn Asn Thr Asn Gly Asp Ser Ser Phe Ser Val Asp
305                 310                 315                 320

Ser Ser Phe Thr Ile Asn Val Ile Gly Gly Ala Thr Ile Gly Val Asn
                325                 330                 335

Ser Pro Thr Val Gly Val Gly Tyr Gln Gly Val Ala Glu Gly Thr Ala
            340                 345                 350

Ile Thr Ile Ser Gly Ile Asn Asn Tyr Glu Leu Val Pro Asn Pro Asp
        355                 360                 365

Leu Gln Lys Asn Leu Pro Met Thr Tyr Gly Thr Cys Asp Pro His Asp
    370                 375                 380

Leu Thr Tyr Ile Lys Tyr Ile Leu Ser Asn Arg Glu Gln Leu Gly Leu
385                 390                 395                 400

Arg Ser Val Met Thr Leu Ala Asp Tyr Asn Arg Met Lys Met Tyr Met
                405                 410                 415

His Val Leu Thr Asn Tyr His Val Asp Glu Arg Glu Ala Ser Ser
            420                 425                 430

<210>  6
<211>  417
<212>  PRT
<213>  Blotched Snakehead virus

<400>  6

Met Asp Phe Ser Lys Glu Asn Thr Gln Ile Arg Tyr Leu Asn Ser Leu
1               5                   10                  15

Leu Val Pro Glu Thr Gly Ser Thr Ser Ile Pro Asp Asp Thr Leu Asp
            20                  25                  30

Arg His Cys Leu Lys Thr Glu Thr Thr Thr Glu Asn Leu Val Ala Ala
        35                  40                  45

Leu Gly Gly Ser Gly Leu Ile Val Leu Phe Pro Asn Ser Pro Ser Gly
    50                  55                  60

Leu Leu Gly Ala His Tyr Thr Lys Thr Pro Gln Gly Ser Leu Ile Phe
65                  70                  75                  80

Asp Lys Ala Ile Thr Thr Ser Gln Asp Leu Lys Lys Ala Tyr Asn Tyr
                85                  90                  95

Ala Arg Leu Val Ser Arg Ile Val Gln Val Arg Ser Ser Thr Leu Pro
            100                 105                 110

Ala Gly Val Tyr Ala Leu Asn Gly Thr Phe Asn Gly Val Thr Tyr Ile
        115                 120                 125

Gly Ser Leu Ser Glu Ile Lys Asp Leu Asp Tyr Asn Ser Leu Leu Ser
    130                 135                 140

Ala Thr Ala Asn Ile Asn Asp Lys Val Gly Asn Val Leu Val Gly Asp
145                 150                 155                 160

Gly Val Ala Val Leu Ser Leu Pro Ala Gly Ser Asp Leu Pro Tyr Val
                165                 170                 175

Arg Leu Gly Asp Glu Val Pro Ser Ser Ala Gly Val Ala Arg Cys Ser
            180                 185                 190

Pro Ser Asp Arg Pro Arg His Tyr Asn Ala Asn Asn Lys Gln Val Gln
        195                 200                 205

Val Gly Thr Thr Asp Thr Lys Thr Asn Gly Phe Asn Ile Asp Ala Thr
    210                 215                 220

Thr Pro Thr Glu Val Thr Val Asp Met Gln Ile Ala Gln Ile Ala Ala
225                 230                 235                 240

Gly Lys Thr Leu Thr Val Thr Val Lys Leu Met Gly Leu Thr Gly Ala
                245                 250                 255

Lys Val Ala Ser Arg Ser Glu Thr Val Ser Gly Asn Gly Gly Thr Phe
            260                 265                 270

His Phe Ser Thr Thr Ala Val Phe Gly Glu Thr Glu Ile Thr Gln Pro
        275                 280                 285

Val Val Gly Val Gln Val Leu Ala Lys Thr Asn Gly Asp Pro Ile Val
    290                 295                 300

Val Asp Ser Tyr Val Gly Val Thr Val His Gly Gly Asn Met Pro Gly
305                 310                 315                 320

Thr Leu Arg Pro Val Thr Ile Ile Ala Tyr Glu Ser Val Ala Thr Gly
                325                 330                 335

Ser Val Leu Thr Leu Ser Gly Ile Ser Asn Tyr Glu Leu Ile Pro Asn
            340                 345                 350

Pro Glu Leu Ala Lys Asn Ile Gln Thr Ser Tyr Gly Lys Leu Asn Pro
        355                 360                 365

Ala Glu Met Thr Tyr Thr Lys Val Val Leu Ser His Arg Asp Glu Leu
    370                 375                 380

Gly Leu Arg Ser Ile Trp Ser Ile Pro Gln Tyr Arg Asp Met Met Ser
385                 390                 395                 400

Tyr Phe Arg Glu Val Ser Asp Arg Ser Ser Pro Leu Lys Ile Ala Gly
                405                 410                 415

Ala

<210>  7
<211>  377
<212>  PRT
<213>  Aliivibrio salmonicida

<400>  7

Val Asn Val Asn Thr Asn Val Ala Ala Met Thr Ala Gln Arg Tyr Leu
1               5                   10                  15

Asn Asn Ala Thr Asn Asp Leu Asn Asn Ser Met Glu Arg Leu Ser Ser
            20                  25                  30

Gly Thr Lys Ile Asn Ser Ala Lys Asp Asp Ala Ala Gly Leu Gln Ile
        35                  40                  45

Ser Asn Arg Leu Thr Ala Gln Thr Arg Gly Leu Asp Val Ala Met Arg
    50                  55                  60

Asn Ala Asn Asp Gly Ile Ser Ile Ala Gln Thr Ala Glu Gly Ala Met
65                  70                  75                  80

Asn Glu Ser Thr Asn Ile Leu Gln Arg Met Arg Asp Leu Ser Leu Gln
                85                  90                  95

Ala Ala Asn Gly Ser Asn Ser Asn Gln Asp Arg Ser Ser Ile Gln Glu
            100                 105                 110

Glu Val His Ala Leu Ser Asp Glu Leu Asn Arg Ile Ala Glu Thr Thr
        115                 120                 125

Ser Phe Gly Gly Arg Arg Leu Leu Asn Gly Gln Phe Gly Glu Ser Ala
    130                 135                 140

Phe Gln Ile Gly Ala Asn Ser Gly Glu Ala Ile Leu Ile Lys Leu Pro
145                 150                 155                 160

Ser Val Arg Ser Asp Asp Asp Ser Met Gly Gly Gln Arg Phe Val Ser
                165                 170                 175

Glu Asn Gly Lys Thr Ala Glu Trp Asn Val Glu Thr Gly Lys Asn Asp
            180                 185                 190

Leu Lys Leu Glu Phe Thr Thr Lys Lys Gly Glu Ala Val Ser Ile Asn
        195                 200                 205

Ile Asn Ala Lys Ala Gly Asp Asp Ile Glu Glu Leu Ala Thr Tyr Ile
    210                 215                 220

Asn Gly Gln Ser Asp Lys Ile Ser Ala Ser Val Gly Asp Glu Gly Lys
225                 230                 235                 240

Leu Gln Leu Phe Val Ala Glu Pro Ser Ile Glu Gly Glu Leu Ala Val
                245                 250                 255

Ser Gly Asn Leu Ala Ser Glu Leu Gly Leu Ala Gly Gly Val Gly Thr
            260                 265                 270

Lys Ala Thr Val Asp Lys Leu Asp Val Thr Ser Val Gly Asp Ala Gln
        275                 280                 285

Val Ser Ile Gly Val Ile Asp Ser Ala Leu Ala Tyr Ile Asp Arg Glu
    290                 295                 300

Arg Ala Asp Leu Gly Ala Lys Gln Asn Arg Leu Gly His Ser Ile Asn
305                 310                 315                 320

Asn Leu Ser Asn Ile Gln Glu Asn Val Ser Ala Ser Asn Ser Arg Ile
                325                 330                 335

Lys Asp Thr Asp Phe Ala Lys Glu Thr Thr Asn Met Thr Lys Ala Gln
            340                 345                 350

Ile Leu Gln Gln Ser Ser Thr Ser Ile Leu Ala Gln Ala Lys Gln Leu
        355                 360                 365

Pro Asn Ala Ala Leu Lys Leu Leu Gln
    370                 375

<210>  8
<211>  24
<212>  DNA
<213>  Artificial

<220>
<223>  CpG oligonucleotide

<400>  8
tcgtcgtttt gtcgttttgt cgtt                                              24


<210>  9
<211>  338
<212>  PRT
<213>  Gallus gallus

<400>  9

Val Cys Arg Val Asp Pro Val Pro Pro Val Ala Pro Glu Val Gln Val
1               5                   10                  15


Leu His Pro Ser Ser Cys Thr Pro Ser Gln Ser Glu Ser Val Glu Leu
            20                  25                  30


Leu Cys Leu Val Thr Gly Phe Ser Pro Ala Ser Ala Glu Val Glu Trp
        35                  40                  45


Leu Val Asp Gly Val Gly Gly Leu Leu Val Ala Ser Gln Ser Pro Ala
    50                  55                  60


Val Arg Ser Gly Ser Thr Tyr Ser Leu Ser Ser Arg Val Asn Val Ser
65                  70                  75                  80


Gly Thr Asp Trp Arg Glu Gly Lys Ser Tyr Ser Cys Arg Val Arg His
                85                  90                  95


Pro Ala Thr Asn Thr Val Val Glu Asp His Val Lys Gly Cys Pro Asp
            100                 105                 110


Gly Ala Gln Ser Cys Ser Pro Ile Gln Leu Tyr Ala Ile Pro Pro Ser
        115                 120                 125


Pro Gly Glu Leu Tyr Ile Ser Leu Asp Ala Lys Leu Arg Cys Leu Val
    130                 135                 140


Val Asn Leu Pro Ser Asp Ser Ser Leu Ser Val Thr Trp Thr Arg Glu
145                 150                 155                 160


Lys Ser Gly Asn Leu Arg Pro Asp Pro Met Val Leu Gln Glu His Phe
                165                 170                 175


Asn Gly Thr Tyr Ser Ala Ser Ser Ala Val Pro Val Ser Thr Gln Asp
            180                 185                 190

Trp Leu Ser Gly Glu Arg Phe Thr Cys Thr Val Gln His Glu Glu Leu
        195                 200                 205

Pro Leu Pro Leu Ser Lys Ser Val Tyr Arg Asn Thr Gly Pro Thr Thr
    210                 215                 220

Pro Pro Leu Ile Tyr Pro Phe Ala Pro His Pro Glu Glu Leu Ser Leu
225                 230                 235                 240

Ser Arg Val Thr Leu Ser Cys Leu Val Arg Gly Phe Arg Pro Arg Asp
                245                 250                 255

Ile Glu Ile Arg Trp Leu Arg Asp His Arg Ala Val Pro Ala Thr Glu
            260                 265                 270

Phe Val Thr Thr Ala Val Leu Pro Glu Glu Arg Thr Ala Asn Gly Ala
        275                 280                 285

Gly Gly Asp Gly Asp Thr Phe Phe Val Tyr Ser Lys Met Ser Val Glu
    290                 295                 300

Thr Ala Lys Trp Asn Gly Gly Thr Val Phe Ala Cys Met Ala Val His
305                 310                 315                 320

Glu Ala Leu Pro Met Arg Phe Ser Gln Arg Thr Leu Gln Lys Gln Ala
                325                 330                 335

Gly Lys

<210>  10
<211>  230
<212>  PRT
<213>  Gallus gallus

<400>  10

Gly Cys Pro Asp Gly Ala Gln Ser Cys Ser Pro Ile Gln Leu Tyr Ala
1               5                   10                  15

Ile Pro Pro Ser Pro Gly Glu Leu Tyr Ile Ser Leu Asp Ala Lys Leu
            20                  25                  30

Arg Cys Leu Val Val Asn Leu Pro Ser Asp Ser Ser Leu Ser Val Thr
        35                  40                  45

Trp Thr Arg Glu Lys Ser Gly Asn Leu Arg Pro Asp Pro Met Val Leu
    50                  55                  60

Gln Glu His Phe Asn Gly Thr Tyr Ser Ala Ser Ser Ala Val Pro Val
65                  70                  75                  80

Ser Thr Gln Asp Trp Leu Ser Gly Glu Arg Phe Thr Cys Thr Val Gln
                85                  90                  95

His Glu Glu Leu Pro Leu Pro Leu Ser Lys Ser Val Tyr Arg Asn Thr
            100                 105                 110

Gly Pro Thr Thr Pro Pro Leu Ile Tyr Pro Phe Ala Pro His Pro Glu
        115                 120                 125

Glu Leu Ser Leu Ser Arg Val Thr Leu Ser Cys Leu Val Arg Gly Phe
    130                 135                 140

Arg Pro Arg Asp Ile Glu Ile Arg Trp Leu Arg Asp His Arg Ala Val
145                 150                 155                 160

Pro Ala Thr Glu Phe Val Thr Thr Ala Val Leu Pro Glu Glu Arg Thr
                165                 170                 175

Ala Asn Gly Ala Gly Gly Asp Gly Asp Thr Phe Phe Val Tyr Ser Lys
            180                 185                 190

Met Ser Val Glu Thr Ala Lys Trp Asn Gly Gly Thr Val Phe Ala Cys
        195                 200                 205

Met Ala Val His Glu Ala Leu Pro Met Arg Phe Ser Gln Arg Thr Leu
    210                 215                 220

Gln Lys Gln Ala Gly Lys
225                 230

<210>  11
<211>  588
<212>  PRT
<213>  Artificial

<220>
<223>  FlagA_HT_ VP2-PD fusion protein

<400>  11

Ala Asp Pro Gly Val Asn Val Asn Thr Asn Val Ala Ala Met Thr Ala
1               5                   10                  15

Gln Arg Tyr Leu Asn Asn Ala Thr Asn Asp Leu Asn Asn Ser Met Glu
            20                  25                  30

Arg Leu Ser Ser Gly Thr Lys Ile Asn Ser Ala Lys Asp Asp Ala Ala
        35                  40                  45

Gly Leu Gln Ile Ser Asn Arg Leu Thr Ala Gln Thr Arg Gly Leu Asp
    50                  55                  60

Val Ala Met Arg Asn Ala Asn Asp Gly Ile Ser Ile Ala Gln Thr Ala
65                  70                  75                  80

Glu Gly Ala Met Asn Glu Ser Thr Asn Ile Leu Gln Arg Met Arg Asp
                85                  90                  95

Leu Ser Leu Gln Ala Ala Asn Gly Ser Asn Ser Asn Gln Asp Arg Ser
            100                 105                 110

Ser Ile Gln Glu Glu Val His Ala Leu Ser Asp Glu Leu Asn Arg Ile
        115                 120                 125

Ala Glu Thr Thr Ser Phe Gly Gly Arg Arg Leu Leu Asn Gly Gln Phe
    130                 135                 140

Gly Glu Ser Ala Phe Gln Ile Gly Ala Asn Ser Gly Glu Ala Ile Leu
145                 150                 155                 160

Ile Lys Leu Pro Ser Val Arg Ser Asp Asp Asp Ser Met Gly Gly Gln
                165                 170                 175

Arg Phe Val Ser Glu Asn Gly Lys Thr Ala Glu Trp Asn Val Glu Thr
            180                 185                 190

Gly Lys Asn Asp Leu Lys Leu Glu Phe Thr Thr Lys Lys Gly Glu Ala
        195                 200                 205

Val Ser Ile Asn Ile Asn Ala Lys Ala Gly Asp Asp Ile Glu Glu Leu
    210                 215                 220

Ala Thr Tyr Ile Asn Gly Gln Ser Asp Lys Ile Ser Ala Ser Val Gly
225                 230                 235                 240

Asp Glu Gly Lys Leu Gln Leu Phe Val Ala Glu Pro Ser Ile Glu Gly
                245                 250                 255

Glu Leu Ala Val Ser Gly Asn Leu Ala Ser Glu Leu Gly Leu Ala Gly
            260                 265                 270

Gly Val Gly Thr Lys Ala Thr Val Asp Lys Leu Asp Val Thr Ser Val
        275                 280                 285

Gly Asp Ala Gln Val Ser Ile Gly Val Ile Asp Ser Ala Leu Ala Tyr
    290                 295                 300

Ile Asp Arg Glu Arg Ala Asp Leu Gly Ala Lys Gln Asn Arg Leu Gly
305                 310                 315                 320

His Ser Ile Asn Asn Leu Ser Asn Ile Gln Glu Asn Val Ser Ala Ser
                325                 330                 335

Asn Ser Arg Ile Lys Asp Thr Asp Phe Ala Lys Glu Thr Thr Asn Met
            340                 345                 350

Thr Lys Ala Gln Ile Leu Gln Gln Ser Ser Thr Ser Ile Leu Ala Gln
        355                 360                 365

Ala Lys Gln Leu Pro Asn Ala Ala Leu Lys Leu Leu Gln Leu Glu Ala
    370                 375                 380

Cys Ala Ser Gly Arg Leu Val Pro Arg Gly Ser Pro Gly Ser Gly Tyr
385                 390                 395                 400

Ile Pro Glu Ala Pro Arg Asp Gly Gln Ala Tyr Val Arg Lys Asp Gly
                405                 410                 415

Glu Trp Val Leu Leu Ser Thr Phe Leu Gly His His His His His His
            420                 425                 430

Asp Tyr Asp Ile Pro Thr Thr Glu Asn Leu Tyr Phe Gln Gly Ala Met
        435                 440                 445

Gly Ser Asp Arg Pro Arg Val Tyr Thr Ile Thr Ala Ala Asp Asp Tyr
    450                 455                 460

Gln Phe Ser Ser Gln Tyr Gln Pro Gly Gly Val Thr Ile Thr Leu Phe
465                 470                 475                 480

Ser Ala Asn Ile Asp Ala Ile Thr Ser Leu Ser Val Gly Gly Glu Leu
                485                 490                 495

Val Phe Arg Thr Ser Val His Gly Leu Val Leu Gly Ala Thr Ile Tyr
            500                 505                 510

Leu Ile Gly Phe Asp Gly Thr Thr Val Ile Thr Arg Ala Val Ala Ala
        515                 520                 525

Asn Asn Gly Leu Thr Thr Gly Thr Asp Asn Leu Met Pro Phe Asn Leu
    530                 535                 540

Val Ile Pro Thr Asn Glu Ile Thr Gln Pro Ile Thr Ser Ile Lys Leu
545                 550                 555                 560

Glu Ile Val Thr Ser Lys Ser Gly Gly Gln Ala Gly Asp Gln Met Ser
                565                 570                 575

Trp Ser Ala Arg Gly Ser Leu Ala Val Thr Ile His
            580                 585

<210>  12
<211>  549
<212>  PRT
<213>  Artificial

<220>
<223>  IgY Fc  nu2-4_HT_P fusion protein

<400>  12

Ala Asp Pro Gly Val Cys Arg Val Asp Pro Val Pro Pro Val Ala Pro
1               5                   10                  15

Glu Val Gln Val Leu His Pro Ser Ser Cys Thr Pro Ser Gln Ser Glu
            20                  25                  30

Ser Val Glu Leu Leu Cys Leu Val Thr Gly Phe Ser Pro Ala Ser Ala
        35                  40                  45

Glu Val Glu Trp Leu Val Asp Gly Val Gly Gly Leu Leu Val Ala Ser
    50                  55                  60

Gln Ser Pro Ala Val Arg Ser Gly Ser Thr Tyr Ser Leu Ser Ser Arg
65                  70                  75                  80

Val Asn Val Ser Gly Thr Asp Trp Arg Glu Gly Lys Ser Tyr Ser Cys
                85                  90                  95

Arg Val Arg His Pro Ala Thr Asn Thr Val Val Glu Asp His Val Lys
            100                 105                 110

Gly Cys Pro Asp Gly Ala Gln Ser Cys Ser Pro Ile Gln Leu Tyr Ala
        115                 120                 125

Ile Pro Pro Ser Pro Gly Glu Leu Tyr Ile Ser Leu Asp Ala Lys Leu
    130                 135                 140

Arg Cys Leu Val Val Asn Leu Pro Ser Asp Ser Ser Leu Ser Val Thr
145                 150                 155                 160

Trp Thr Arg Glu Lys Ser Gly Asn Leu Arg Pro Asp Pro Met Val Leu
                165                 170                 175

Gln Glu His Phe Asn Gly Thr Tyr Ser Ala Ser Ser Ala Val Pro Val
            180                 185                 190

Ser Thr Gln Asp Trp Leu Ser Gly Glu Arg Phe Thr Cys Thr Val Gln
        195                 200                 205

His Glu Glu Leu Pro Leu Pro Leu Ser Lys Ser Val Tyr Arg Asn Thr
    210                 215                 220

Gly Pro Thr Thr Pro Pro Leu Ile Tyr Pro Phe Ala Pro His Pro Glu
225                 230                 235                 240

Glu Leu Ser Leu Ser Arg Val Thr Leu Ser Cys Leu Val Arg Gly Phe
                245                 250                 255

Arg Pro Arg Asp Ile Glu Ile Arg Trp Leu Arg Asp His Arg Ala Val
            260                 265                 270

Pro Ala Thr Glu Phe Val Thr Thr Ala Val Leu Pro Glu Glu Arg Thr
        275                 280                 285

Ala Asn Gly Ala Gly Gly Asp Gly Asp Thr Phe Phe Val Tyr Ser Lys
    290                 295                 300

Met Ser Val Glu Thr Ala Lys Trp Asn Gly Gly Thr Val Phe Ala Cys
305                 310                 315                 320

Met Ala Val His Glu Ala Leu Pro Met Arg Phe Ser Gln Arg Thr Leu
                325                 330                 335

Gln Lys Gln Ala Gly Lys Leu Glu Ala Cys Ala Ser Gly Arg Leu Val
            340                 345                 350

Pro Arg Gly Ser Pro Gly Ser Gly Tyr Ile Pro Glu Ala Pro Arg Asp
        355                 360                 365

Gly Gln Ala Tyr Val Arg Lys Asp Gly Glu Trp Val Leu Leu Ser Thr
    370                 375                 380

Phe Leu Gly His His His His His His Asp Tyr Asp Ile Pro Thr Thr
385                 390                 395                 400

Glu Asn Leu Tyr Phe Gln Gly Ala Met Gly Ser Asp Arg Pro Arg Val
                405                 410                 415

Tyr Thr Ile Thr Ala Ala Asp Asp Tyr Gln Phe Ser Ser Gln Tyr Gln
            420                 425                 430

Pro Gly Gly Val Thr Ile Thr Leu Phe Ser Ala Asn Ile Asp Ala Ile
        435                 440                 445

Thr Ser Leu Ser Val Gly Gly Glu Leu Val Phe Arg Thr Ser Val His
    450                 455                 460

Gly Leu Val Leu Gly Ala Thr Ile Tyr Leu Ile Gly Phe Asp Gly Thr
465                 470                 475                 480

Thr Val Ile Thr Arg Ala Val Ala Ala Asn Asn Gly Leu Thr Thr Gly
                485                 490                 495

Thr Asp Asn Leu Met Pro Phe Asn Leu Val Ile Pro Thr Asn Glu Ile
            500                 505                 510

Thr Gln Pro Ile Thr Ser Ile Lys Leu Glu Ile Val Thr Ser Lys Ser
        515                 520                 525

Gly Gly Gln Ala Gly Asp Gln Met Ser Trp Ser Ala Arg Gly Ser Leu
    530                 535                 540

Ala Val Thr Ile His
545

<210>  13
<211>  439
<212>  PRT
<213>  Artificial

<220>
<223>  IgY Fc nu3-4_HT_P fusion protein

<400>  13

Ala Asp Pro Gly Cys Pro Asp Gly Ala Gln Ser Cys Ser Pro Ile Gln
1               5                   10                  15

Leu Tyr Ala Ile Pro Pro Ser Pro Gly Glu Leu Tyr Ile Ser Leu Asp
            20                  25                  30

Ala Lys Leu Arg Cys Leu Val Val Asn Leu Pro Ser Asp Ser Ser Leu
        35                  40                  45

Ser Val Thr Trp Thr Arg Glu Lys Ser Gly Asn Leu Arg Pro Asp Pro
    50                  55                  60

Met Val Leu Gln Glu His Phe Asn Gly Thr Tyr Ser Ala Ser Ser Ala
65                  70                  75                  80

Val Pro Val Ser Thr Gln Asp Trp Leu Ser Gly Glu Arg Phe Thr Cys
                85                  90                  95

Thr Val Gln His Glu Glu Leu Pro Leu Pro Leu Ser Lys Ser Val Tyr
            100                 105                 110

Arg Asn Thr Gly Pro Thr Thr Pro Pro Leu Ile Tyr Pro Phe Ala Pro
        115                 120                 125

His Pro Glu Glu Leu Ser Leu Ser Arg Val Thr Leu Ser Cys Leu Val
    130                 135                 140

Arg Gly Phe Arg Pro Arg Asp Ile Glu Ile Arg Trp Leu Arg Asp His
145                 150                 155                 160

Arg Ala Val Pro Ala Thr Glu Phe Val Thr Thr Ala Val Leu Pro Glu
                165                 170                 175

Glu Arg Thr Ala Asn Gly Ala Gly Gly Asp Gly Asp Thr Phe Phe Val
            180                 185                 190

Tyr Ser Lys Met Ser Val Glu Thr Ala Lys Trp Asn Gly Gly Thr Val
        195                 200                 205

Phe Ala Cys Met Ala Val His Glu Ala Leu Pro Met Arg Phe Ser Gln
    210                 215                 220

Arg Thr Leu Gln Lys Gln Ala Gly Lys Leu Glu Ala Cys Ala Ser Gly
225                 230                 235                 240

Leu Val Pro Arg Gly Ser Pro Gly Ser Gly Tyr Ile Pro Glu Ala Pro
                245                 250                 255

Arg Asp Gly Gln Ala Tyr Val Arg Lys Asp Gly Glu Trp Val Leu Leu
            260                 265                 270

Ser Thr Phe Leu Gly His His His His His His Asp Tyr Asp Ile Pro
        275                 280                 285

Thr Thr Glu Asn Leu Tyr Phe Gln Gly Ala Met Gly Ser Asp Arg Pro
    290                 295                 300

Arg Val Tyr Thr Ile Thr Ala Ala Asp Asp Tyr Gln Phe Ser Ser Gln
305                 310                 315                 320

Tyr Gln Pro Gly Gly Val Thr Ile Thr Leu Phe Ser Ala Asn Ile Asp
                325                 330                 335

Ala Ile Thr Ser Leu Ser Val Gly Gly Glu Leu Val Phe Arg Thr Ser
            340                 345                 350

Val His Gly Leu Val Leu Gly Ala Thr Ile Tyr Leu Ile Gly Phe Asp
        355                 360                 365

Gly Thr Thr Val Ile Thr Arg Ala Val Ala Ala Asn Asn Gly Leu Thr
    370                 375                 380

Thr Gly Thr Asp Asn Leu Met Pro Phe Asn Leu Val Ile Pro Thr Asn
385                 390                 395                 400

Glu Ile Thr Gln Pro Ile Thr Ser Ile Lys Leu Glu Ile Val Thr Ser
                405                 410                 415

Lys Ser Gly Gly Gln Ala Gly Asp Gln Met Ser Trp Ser Ala Arg Gly
            420                 425                 430

Ser Leu Ala Val Thr Ile His
        435

<210>  14
<211>  128
<212>  PRT
<213>  Artificial

<220>
<223>  Monomeric avidin

<400>  14

Ala Arg Lys Cys Ser Leu Thr Gly Lys Trp Thr Asn Asp Leu Gly Ser
1               5                   10                  15

Asn Met Thr Ile Gly Ala Val Asn Ser Arg Gly Glu Phe Thr Gly Thr
            20                  25                  30

Tyr Ile Thr Ala Val Thr Ala Thr Ser Asn Glu Ile Lys Glu Ser Pro
        35                  40                  45

Leu His Gly Thr Gln Ala Thr Ile Asn Lys Arg Thr Gln Pro Thr Phe
    50                  55                  60

Gly Phe Thr Val Asn Trp Lys Phe Ser Glu Ser Thr Thr Val Phe Thr
65                  70                  75                  80

Gly Gln Cys Phe Ile Asp Arg Asn Gly Lys Glu Val Leu Lys Thr Met
                85                  90                  95

Trp Leu Leu Arg Ser Ser Val Asn Asp Ile Gly Asp Asp Lys Lys Ala
            100                 105                 110

Thr Arg Val Gly Ile Asn Ile Phe Thr Arg Leu Arg Thr Gln Lys Glu
        115                 120                 125

<210>  15
<211>  340
<212>  PRT
<213>  Artificial

<220>
<223>  P_HT_mAv fusion protein

<400>  15

Ala Asp Pro Gly Ser Asp Arg Pro Arg Val Tyr Thr Ile Thr Ala Ala
1               5                   10                  15

Asp Asp Tyr Gln Phe Ser Ser Gln Tyr Gln Pro Gly Gly Val Thr Ile
            20                  25                  30

Thr Leu Phe Ser Ala Asn Ile Asp Ala Ile Thr Ser Leu Ser Val Gly
        35                  40                  45

Gly Glu Leu Val Phe Arg Thr Ser Val His Gly Leu Val Leu Gly Ala
    50                  55                  60

Thr Ile Tyr Leu Ile Gly Phe Asp Gly Thr Thr Val Ile Thr Arg Ala
65                  70                  75                  80

Val Ala Ala Asn Asn Gly Leu Thr Thr Gly Thr Asp Asn Leu Met Pro
                85                  90                  95

Phe Asn Leu Val Ile Pro Thr Asn Glu Ile Thr Gln Pro Ile Thr Ser
            100                 105                 110

Ile Lys Leu Glu Ile Val Thr Ser Lys Ser Gly Gly Gln Ala Gly Asp
        115                 120                 125

Gln Met Ser Trp Ser Ala Arg Gly Ser Leu Ala Val Thr Ile His Gly
    130                 135                 140

Gly Leu Glu Ala Cys Ala Ser Gly Arg Leu Val Pro Arg Gly Ser Pro
145                 150                 155                 160

Gly Ser Gly Tyr Ile Pro Glu Ala Pro Arg Asp Gly Gln Ala Tyr Val
                165                 170                 175

Arg Lys Asp Gly Glu Trp Val Leu Leu Ser Thr Phe Leu Gly His His
            180                 185                 190

His His His His Asp Tyr Asp Ile Pro Thr Thr Glu Asn Leu Tyr Phe
        195                 200                 205

Gln Gly Ala Met Ala Arg Lys Cys Ser Leu Thr Gly Lys Trp Thr Asn
    210                 215                 220

Asp Leu Gly Ser Asn Met Thr Ile Gly Ala Val Asn Ser Arg Gly Glu
225                 230                 235                 240

Phe Thr Gly Thr Tyr Ile Thr Ala Val Thr Ala Thr Ser Asn Glu Ile
                245                 250                 255

Lys Glu Ser Pro Leu His Gly Thr Gln Ala Thr Ile Asn Lys Arg Thr
            260                 265                 270

Gln Pro Thr Phe Gly Phe Thr Val Asn Trp Lys Phe Ser Glu Ser Thr
        275                 280                 285

Thr Val Phe Thr Gly Gln Cys Phe Ile Asp Arg Asn Gly Lys Glu Val
    290                 295                 300

Leu Lys Thr Met Trp Leu Leu Arg Ser Ser Val Asn Asp Ile Gly Asp
305                 310                 315                 320

Asp Lys Lys Ala Thr Arg Val Gly Ile Asn Ile Phe Thr Arg Leu Arg
                325                 330                 335

Thr Gln Lys Glu
            340

## Description

### FIELD OF THE INVENTION

The invention relates to immunogenic compositions useful for inducing an immune response towards a birnavirus infection by means of the use of antigenic regions of the birnavirus VP2 polypeptide as well as to the use of conjugates comprising said VP2 polypeptide and an immune co-stimulatory molecule.

### BACKGROUND OF THE INVENTION

Infectious bursal disease (IBD), or Gumboro disease, is an acute-course viral disease which mainly affects industrially fattened chickens (broilers) in a growth stage (3-8 weeks). This disease is caused by the infectious bursal disease virus (IBDV), also known as the Gumboro disease virus, having a very pronounced tropism by the lymphoid cells located in the bursa of Fabricius, giving rise to a selective elimination of the B- lymphocyte precursors present in the bursa of Fabricius, which leads to an immunosuppression of the affected animals. The severity of the disease depends both on the degree of virulence of the viral strain and on the immunological state of the infected animal and its clinical consequences range between establishing a transitory immunosuppression, which essentially means a reduction in growth rates and a decrease in the capacity of response against infection and vaccination against other avian pathogens, and a severe immunosuppression leading to the death of a high percentage of the infected animals. Therefore, the infection by IBDV causes high mortality rates due to two causes: i) the infective IBDV cycle itself, and ii) opportunist infections of other pathogens affecting immunodepressed animals. The immunosuppressant effect of IBDV also decreases the response of the animals to vaccines against other avian pathogens.

The economic importance of the disease mainly lies in these two aspects: on the one hand, the high mortality produced by several IBDV strains in chickens of 3 weeks of age or even more and, on the other hand, the second clinical indication of the disease consisting of a prolonged immunosuppression of infected birds in early ages. The main after effects associated to said immunosuppression are: gangrenous dermatitis, anemia-hepatitis syndrome with inclusion bodies, E. coli infections and failures in the efficacy of other vaccinations such as those against Newcastle disease and infectious bronchitis.

The virus is transmitted through water, food or excrements, but there is no vertical transmission through eggs and there are no chronic carriers of this disease. Therefore, the success in IBD control lies in the application of strict hygiene and installation disinfection programs together with the preventive vaccination of breeders and offspring. The immunization of breeding birds is especially important for the transmission of passive immunity to the offspring; nevertheless, the presence of said passive immunity can interfere in the efficacy of the offspring vaccination.

The vaccination programs against IBDV involve a double action: i) vaccination of laying hens; and ii) vaccination of broilers. The laying hens receive an inactivated vaccine dose at 16 or 20 weeks of age, followed by one or more vaccinations with an attenuated live vaccine at different time intervals. The basic purpose of this vaccination is the transmission to descendants of maternal antibodies capable of neutralizing the ineffectiveness of the virus for the first 3-4 weeks of the life of the chicken through the yolk sac. From 2 weeks of life onwards, the chickens are vaccinated (with one or several doses) by means of attenuated live vaccine administration in drinking water.

IBDV is the prototype of the Avibirnavirus genus of the Birnaviridae family, a double-stranded RNA virus (dsRNA). The IBDV virions have a size of approximately 70 nm (60-65 nm), do not have lipid coating and have an icosahedral symmetry. The genome has an encoding capacity for five proteins VP1, VP2, VP3, VP4 and VP5. The capsid is formed by the VP2 protein whereas the VP3 and VP1 polypeptides (the latter has RNA polymerase activity) are found associated to the RNA of the virion.

The serological response to the virus is mainly directed to the VP2 protein. In fact, most monoclonal antibodies capable of neutralizing the ineffectiveness of IBDV recognize conformational epitopes in the VP2 protein and are therefore neutralizing antibodies. Vaccines containing attenuated live IBDV (US 5,632,989) and vaccines with inactivated IBDV capable of producing protection against field strains have been described.

However, the administration of live birnavirus vaccines may result in the replication of vaccine viruses in vaccinated hosts, affecting the natural maturation of their immune system and reducing its ability to respond to other vaccines. Moreover, vaccine virus replication in vaccinated animals results in the release of large quantities of infectious virus to environment and might promote the establishment of persistent infections in vaccinated specimens and, lastly, the immune response generated these vaccines do not allow the discrimination between infected and vaccinated animals.

The use of vaccines containing live recombinant viruses also raises bio-safety concerns. The most important one concerning the arousal and environmental release of new virus strains generated by recombination between the genomes of vaccine and field viruses or by mutations on the recombinant virus genome.

VLPs constitute an alternative to the use of attenuated live vaccines and of recombinant subunit vaccines. VLPs are obtained by the self- assembly of the subunits forming the viral capsid and mimic the structure and antigenic properties of the native virion although they lack genetic material so they are incapable of replicating. recombinant subunit vaccines containing the VP2 protein of IBDV expressed in various systems, for example, bacteria, yeasts, virus, etc, normally in the form of fusion proteins have been described (US 5,605,792, US 5,605,827). On the other hand, the possibility that VP2 forms VLPs in combination with the entire or part of the VP3 of IBDV is known (US 5,788,970, Hu et al., 1999, Biotechnology and Bioengineering, 63(6), 721-729; Wang et al., 2000, Biotechnology and Bioengineering, 67(1), 104-111; Martinez-Torrecuadrada et al., 2000a, Clin. Diagn. Lab. Immunol., 7 (4): 645-651; Martinez-Torrecuadrada et al., 2000b, Virology 278:322-331; Cheng et al., 2001 Biotechnol. Prog., 17, 318-325). Likewise, the obtaining and use of VLPs of IBDV as vaccines have been described, see, for example, US patent 5,788,970, patent publications WO2004087900, WO2005/071068, WO2005/071069 and WO2005/105834, Spanish patent application P200501733, etc. However, vaccines based on VLPs require expensive production systems; show poor long-term stability and usually require the presence of adjuvants for being efficacious. This not only increases the costs of the vaccine but also might cause unwanted cytotoxic and inflammatory side-effects.

Immunogenic compositions capable of inducing an immune response towards IBDV have been obtained by forming an immune complex by mixing a certain amount of specific antibodies obtained from the serum of hyperimmunized chickens with live infectious bursal disease vaccine virus (IBDV) (Jeurissen et al, 1998, Immunology, 95:494-500). However, these type of vaccines raises bio-safety concerns due, in particular, to the arousal and environmental release of new virus strains generated by recombination between the genomes of vaccine and field viruses or by mutations on the recombinant virus genome. Moreover, the vaccines based on immune complex are expensive to produce and their formulation poses technical problems specially those concerning batch-to-batch reproducibility.

Thus, there is still a need for vaccines specifically directed towards the immunodominant viral antigen and which overcome the drawbacks of the vaccines known to date.

### SUMMARY OF THE INVENTION

It has now been observed, that, surprisingly, a conjugate comprising the PD domain of IBDV VP2 and lacking the AA' hairpin region of the PD domain, optionally coupled to a region capable of targeting said VP2 PD domain to cells of the immune system, allows the production of a vaccine capable of inducing innate and adaptive immune responses, which results in immune responses exclusively focused to a critical virus protein domain (VP2-PD), thus avoiding the immune-dominance effect of irrelevant virus polypeptides or polypeptide domains. This conjugate is suitable for the development of vaccines which are non-infectious, do not require adjuvants, require lower costs for their production, show less adverse vaccine side-effects and is amenable for lyophilization, thus providing long-term vaccine stability.

### LEGENDS TO THE FIGURES

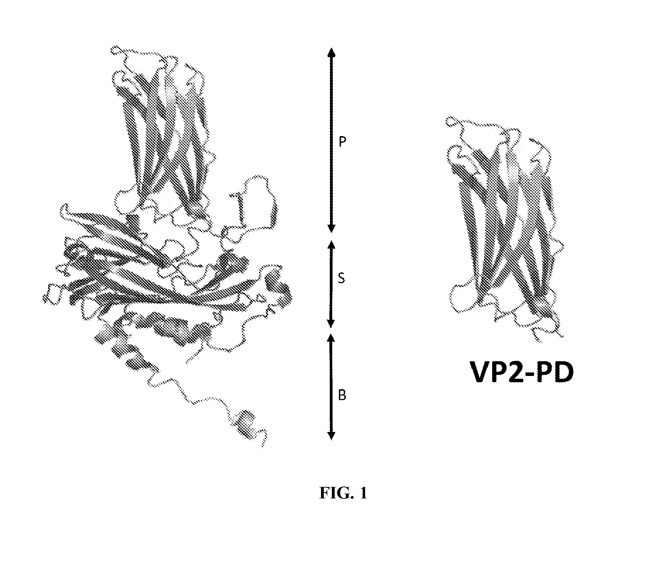

**Figure 1** is a representation of the topology of the projection domain of VP2 (left) and of the VP2 projection domain. Arrows indicate β sheets and ribbons indicate α-helixes. P, S and B indicate, respectively, the projection domain, the shell domain and the base domain.

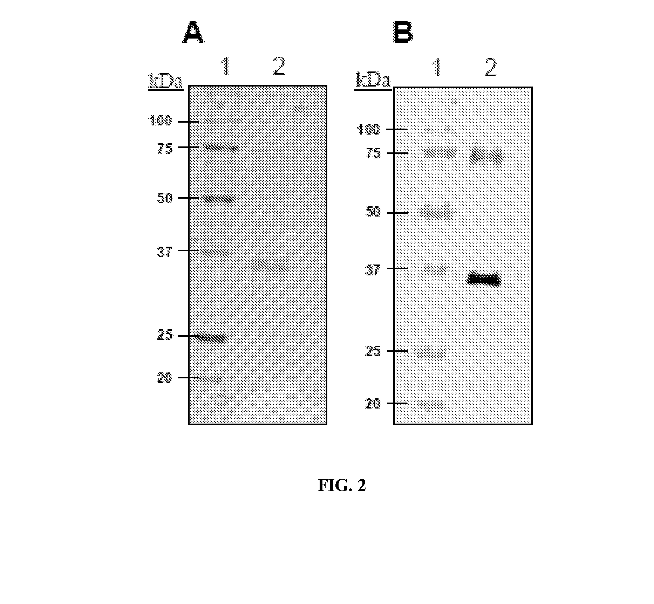

**Figure 2** **A:** SDS-PAGE of the purified P_HT_mAV stained with coomassie. **B:** Western-blot of the purified protein detected with Rabbit Anti-avidin polyclonal antibody (1:1000) followed by Peroxidase conjugated Goat Anti-Rabbit antibody (1:5000). Lane 1: Molecular weight standards; lane 2: Purified protein.

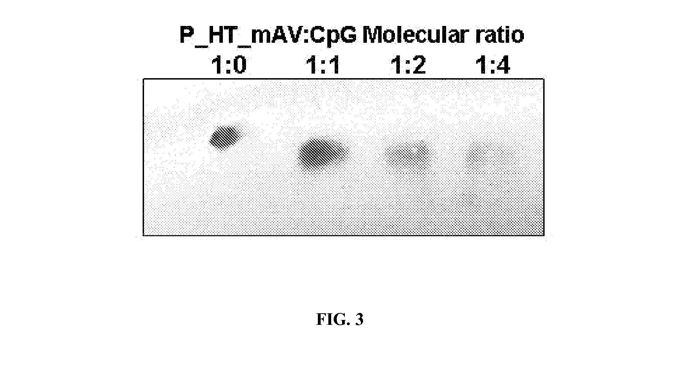

**Figure 3****:** Native electrophoresis of conjugates formed by combination of P_HT_mAV and biotinylated CpG at different ratios in a 0.7 % agarose gel transferred to nitrocellulose. Native protein was detected with a Rabbit Anti-VP2 polyclonal antibody (1:1000) and Peroxidase-conjugated Goat Anti-Rabbit antibody (1:10000).

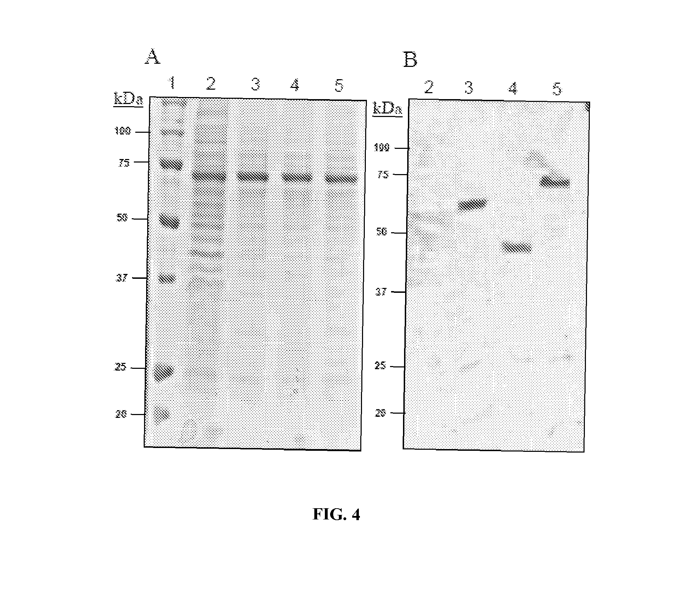

**Figure 4****. A.** SDS_PAGE of culture medium of insect cells infected with different recombinant baculoviruses, stained with coomassie. The main band corresponds to the BSA present in the culture medium. **B.** Western-blot of the same samples, revealed with Rabbit Anti-VP2 polyclonal antibody (1:1000) followed by Peroxidase conjugated Goat Anti-Rabbit (1:5000). Lane 1: Molecular weight standards; Lane 2: Mock-infected cells; Lane 3: IgY Fc υ2-4_HT_P; Lane 4: IgY Fc υ3-4_HT_P; Lane 5: FlagA_HT_P.

### DETAILED DESCRIPTION OF THE INVENTION

### 1. Polypeptides of the invention

In a first aspect, the invention relates to a polypeptide comprising a birnavirus VP2 P domain lacking the AA' hairpin region.

As used herein, the term "polypeptide" refers to a polymer in which the monomers are amino acids and are joined together through peptide or disulphide bonds.

As used herein, the term "birnavirus" refers to any virus belonging to the birnaviridae family which are are type-III viruses in the Baltimore classification, which means they have a double-stranded RNA genome. The birnaviridae family includes the genera Aquabirnavirus (e.g., Infectious pancreatic necrosis virus); the Avibirnavirus (e.g. Infectious bursal disease virus); the Blosnavirus (e.g. Blotched snakehead virus) and the Entomobirnavirus (e.g. Drosophila X virus).

In a preferred embodiment, the birnavirus is an avibirnavirus. In a still more preferred embodiment, the avibirnavirus is IBDV. The term "infectious bursal disease virus" (IBDV), as used herein, encompasses all IBDV strains and all serotypes and variants thereof, including live, attenuated, killed or otherwise inactivated forms belonging to any of the known serotypes (1 or 2) [by way of illustration see the review by van den Berg TP et al. (2000)].

In another preferred embodiment, the birnavirus is an aquavirnavirus. In a still more preferred embodiment, the aquavirnavirus is IPNV. The term "infectious pancreatic necrosis virus" (IPNV), as used herein, encompasses all IPNV strains and all serotypes and variants thereof, including live, attenuated, killed or otherwise inactivated forms belonging to any of the known serotypes.

As used herein, the term "birnavirus VP2 (or pVP2)" generally relates to a protein the amino acid sequence of which comprises or is formed by the amino acid sequence of the birnavirus VP2 of any birnavirus as well as proteins that are substantially homologous to said IBDV proteins pVP2, i.e. proteins having a good alignment with the sequence of a certain IBDV pVP2, for example, proteins the amino acid sequences of which have a degree of freedom with respect to said IBDV proteins pVP2 of at least 60%, preferably of at least 80%, more preferably of at least 90% and even more preferably of at least 95%. Sequences that are homologous to a sequence of IBDV protein pVP2 can be easily identified a person skilled in the art with the aid of a suitable computer program for comparing sequences, for example, the BLAST program (Altschul et al. (1997)). In a particular embodiment, IBDV protein pVP2 is the Soroa strain IBDV protein pVP2 the amino acid sequence of which, complete length, is deposited in the NCBI with accession number AAD30136. VP2 proteins found in different IBDV strains have been reported to present a protein sequence homology of over 80 percent. VP2 proteins of other Birnaviridae share homologies with IBDV of 40 percent for aquatic Birnavirus and 30 percent for Drosophila Birnavirus [Coulibaly F. et al., Cell, 2005, 120:761-772]. The IBDV VP2 preprotein comprises amino acids 1 ― 512 of the VP2-3-4 polyprotein as defined in UniProt accession number Q82635 (Version 47 of 11 January 2011). The mature VP2 comprises amino acids 1 ― 441 of the VP2-3-4 polyprotein as defined in UniProt accession number Q82635 (Version 47 of 11 January 2011).

The term "P domain", used herein indistinctly with "projection domain" or "PD" refers to a β barrel with jelly ribbon topology and which comprises the β-strands B, C, D, E, F, G, H and I (see figure 1). In a preferred embodiment, the P domain comprises the sequence of α-helixes B, C, D, E, F and G and the interconnecting loops BC, CD, DE, EF and FG. Preferably, the polypeptide of the invention lacks the VP2 shell (S) domain and/or the VP2 base (B) domain (see figure 1)

In a still more preferred embodiment, the P domain is from IBDV and comprises the sequence: 
1   SDRPRVYTIT  AADDYQFSSQ  YQPGGVTITL  FSANIDAITS  LSVGGELVFR

51   TSVHGLVLGA  TIYLIGFDGT  TVITRAVAAN  NGLTTGTDNL  MPFNLVIPTN

101  EITQPITSIK  LEIVTSKSGG  QAGDQMSWSA  RGSLAVTIHG  G (SEQ ID NO:1)
 or the sequence 
1 SDRPRVYTIT AADDYQFSSQ YQPGGVTITL FSANIDAITS LSVGGELVFR

51 TSVHGLVLGA TIYLIGFDGT TVITRAVAAN NGLTTGTDNL MPFNLVIPTN

101 EITQPITSIK LEIVTSKSGG QAGDQMSWSA RGSLAVTIH (SEQ ID NO:2)
 wherein the underlined regions correspond, respectively from the N- to the C-terminus, to the B, C, D, E, F, G, H and I β-strands.

The corresponding P domain sequences in VP2 proteins of the viruses of the birnaviridae family can be determined by identifying regions showing substantial sequence similarity with the P domain sequences in IBDV VP2 using any tool available for determining sequence identity.

For the purpose of the present invention, sequence "identity" is objectively determined by any of a number of methods. The skilled person is well aware of these methods and can choose a suitable method without undue burden. A variety of methods for determining relationships between two or more sequences (e.g. identity, similarity and/or homology) are available and well known in the art. The methods include manual alignment, computer assisted sequence alignment and combinations thereof, for example. A number of algorithms (which are generally computer implemented) for performing sequence alignment are widely available or can be produced by one of skill. These methods include, e.g. the local homology algorithm of Smith and Waterman (1981) Adv. Appl. Math. 2: 482; the homology algorithm of Needleman and Wunsch (1970) J. MoI. Biol. 48: 443; the search for similarity method of Pearson and Lipman (1988) Proc. Natl. Acad. Sci. (USA) 85: 2444; and/or by computerized implementations of these algorithms (e.g. GAP, BESTFIT, FASTA, and TFASTA in the Wisconsin Genetics Software Package Release 7.0, Genetics Computer Group, 575 Science Dr., Madison, Wl). For example, software for performing sequence identity (and sequence similarity) analysis using the BLAST algorithm is described in Altschul et al. (1990) J. MoI. Biol. 215: 403-410. This software is publicly available, e.g. through the National Center for Biotechnology Information on the World Wide Web at ncbi.nlm.nih.gov. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighbourhood word score threshold. These initial neighbourhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and, speed of the alignment. The BLASTN program (for nucleotide sequences) uses as defaults a word length (W) of 11, an expectation (E) of 10, a cutoff of 100, M=5, N=-4, and a comparison of both strands. For amino acid sequences, the BLASTP (BLAST Protein) program uses as defaults a word length (W) of 3, an expectation (E) of 10, and the BLOSUM62 scoring matrix (see, Henikoff and Henikoff (1989) Proc. Natl. Acad. Sci. USA 89: 10915).

Additionally, the BLAST algorithm performs a statistical analysis of the similarity between two sequences (see, e.g. Karlin and Altschul (1993) Proc. Natl. Acad. Sci.USA 90: 5873-5787). One measure of similarity provided by the BLAST algorithm is the smallest sum probability (p (N)), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0. 01, and or even less than about 0.001.

Another example of a useful sequence alignment algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng and Doolittle (1987) J. MoI. Evol. 35: 351 -360. The method used is similar to the method described by Higgins and Sharp (1989) CABIOS5: 151 -153. The program can align, e.g. up to 300 sequences of a maximum length of 5,000 letters. The multiple alignment procedure begins with the pairwise alignment of the two most similar sequences, producing a cluster of two aligned sequences. This cluster can then be aligned to the next most related sequence or cluster of aligned sequences. Two clusters of sequences can be aligned by a simple extension of the pairwise alignment of two individual sequences. The final alignment is achieved by a series of progressive, pairwise alignments. The program can also be used to plot a dendogram or tree representation of clustering relationships. The program is run by designating specific sequences and their amino acid or nucleotide coordinates for regions of sequence comparison. An additional example of an algorithm that is suitable for multiple DNA, or amino acid, sequence alignments is the CLUSTALW program (Thompson, J. D. et al. (1994) Nucl. Acids. Res. 22: 4673-4680). CLUSTALW performs multiple pairwise comparisons between groups of sequences and assembles them into a multiple alignment based on homology. Gap open and Gap extension penalties can be, e. g., 10 and 0.05 respectively. For amino acid alignments, the BLOSUM algorithm can be used as a protein weight matrix. See, e. g., Henikoff and Henikoff (1992) Proc. Natl. Acad. Sci. USA 89: 10915-10919.

The alignment of the sequences of different viruses of the birnaviridae family using the CLUSTALW algorithm results in the following multiple sequence alignment. 
CLUSTAL 2.1 multiple sequence alignment

IBDV            MTNLQDQTQQIVPFIRSLLMPTTGPASIPDDTLEKHTLRSETSTYNLTVGDTGSGLIVFF 60
BSNV            -MDFSKENTQIR-YLNSLLVPETGSTSIPDDTLDRHCLKTETTTENLVAALGGSGLIVLF 58
IPNV            ----MNTNKATATYLKSIMLPETGPASIPDDITERHILKQETSSYNLEVSESGSGVLVCF 56
DRXV            -------MNTTNEYLKTLLNPAQFISDIPDDIMIRHVNSAQTITYNLKSGASGTGLIVVY 53
                          ::.::: *    :.****   :*    :* : **  .  *:*::* :

IBDV            PGFPGSIVGAHYTLQSNGN-YKFDQMLLTAQNLPASYNYCRLVSRSLTVRSSTLPGGVYA 119
BSNV            PNSPSGLLGAHYTKTPQGS-LIFDKAITTSQDLKKAYNYARLVSRIVQVRSSTLPAGVYA 117
IPNV            PGAPGSRIGAHYRWNANQTGLEFDQWLETSQDLKKAFNYGRLISRKYDIQSSTLPAGLYA 116
DRXV            PNTPSSISGFHYIWDSATSNWVFDQYIYTAQELKDSYDYGRLISGSLSIKSSTLPAGVYA 113
                *. *..   * **    .   .   **: : *:*:*  :::* **:*    ::*****.*:**

IBDV            LNGTINAVTFQGSLSELTDVSYNGLMSATANINDKIGNVLVGEGVTVLSLPTSYDLGYVR 179
BSNV            LNGTFNGVTYIGSLSEIKDLDYNSLLSATANINDKVGNVLVGDGVAVLSLPAGSDLPYVR 177
IPNV            LNGTLNAATFEGSLSEVESLTYNSLMSLTTNPQDKVNNQLVTKGVTVLNLPTGFDKPYVR 176
DRXV            LNGTFNAVWFQGTLSEVSDYSYDRILSITSNPLDKVGNVLVGDGIEVLSLPQGFNNPYVR 173
                ****:*..  : *:***:  .   *: ::* *:*   **:.* ** .*: **.**  .  :   ***

IBDV            LGDPIPAIGLDP-KMVATCDSSDRPRVYTITAADDYQFSSQYQPGGVTITLFSANIDAIT 238
BSNV            LGDEVPSS-----AGVARCSPSDRPRHYNAN-------NKQVQVGTTDTKTNGFNIDATT 225
IPNV            LEDETPQGLQSMNGAKMRCTAAIAPRRYEIDLPS--QRLPPVPATGTLTTLYEGNADIVN 234
DRXV            LGDKSPSTLSSPTHITNTSQNLATGGAYMIP-------VTTVPGQGFHNKEFSINVDSVG 226
                * *  *              .          *                    .    * *

IBDV            SLSVGGELVF----QTSVHGLVLGATIYLIGFDG-TAVITRAVAANNGLTTGTDNLLPFN 293
BSNV            PTEVTVDMQI----AQIAAGKTLTVTVKLMGLTG-AKVASRSETVSG---NGGTFHFSTT 277
IPNV            STTVTGDINFSLA-EQPADETKFDFQLDFMGLDNDVPVVTVVSSVLATNDNYRGVSAKMT 293
 
DRXV PVDILWSGQMTMQDEWTVTANYQPLNISGTLIANSQRTLTWSNTGVSNGSHYMNMNNLNV 286
. : . : . : : . . : :

IBDV LVIPTNEITQPITSIKLEIVTSKSGGQAGDQMSWSARGSLAVTIHGG--NYPGALRPVTL 351
BSNV AVFGETEITQPVVGVQVLAKTN------GDPIVVDS--YVGVTVHGG--NMPGTLRPVTI 327
IPNV QSIPTENITKPITRVKLSYKIN-QQTAIGNVATLGTMGPASVSFSSGNGNVPGVLRPITL 352
DRXV SLFHENPPPEPVAAIKININYG---NNTNGDSSFSVDSSFTINVIGG--ATIGVNSPTVG 341
: .:*:. ::: . .. . :.. .* *. * .

IBDV VAYERVATGSVVTVAGVSNFELIPNPELAKNLVTEYGRFDPGAMNYTKLILSERDRLGIK 411
BSNV IAYESVATGSVLTLSGISNYELIPNPELAKNIQTSYGKLNPAEMTYTKVVLSHRDELGLR 387
IPNV VAYEKMTPLSILTVAGVSNYELIPNPELLKNMVTRYGKYDPEGLNYAKMILSHREELDIR 412
DRXV VGYQGVAEGTAITISGINNYELVPNPDLQKNLPMTYGTCDPHDLTYIKYILSNREQLGLR 401
:.*: :: : :*::*:.*:**:***:* **: ** :* :.* * :**.*:.*.::

IBDV TVWPTREYTDFREYFMEVADLNSPLKIAGA 441 (SEQ ID NO:3)
BSNV SIWSIPQYRDMMSYFREVSDRSSPLKIAGA 417 (SEQ ID NO:4)
IPNV TVWRTEEYKERTRVFNEITDFSSDLPTSKA 442 (SEQ ID NO:5)
DRXV SVMTLADYNRMKMYMHVLTNYHVDEREASS 431 (SEQ ID NO:6)
:: :* : ::: : :
 wherein the highlighted region corresponds to the P domain lacking the AA' hairpin region.

The term "AA' hairpin", as used herein, refers to a β hairpin structure which forms a flap that invades and complements the β barrel of the neighbouring P domain.

The term β hairpin, as used herein, refers to a protein motif consisting of two beta strands that are adjacent in primary structure oriented in an antiparallel arrangement (where the N-terminus of one sheet is adjacent to the C-terminus of the next) and linked by a short loop of two to five amino acids.

In a preferred embodiment, the AA' hairpin is that of IBDV VP2 consisting of amino acids 181 to 200. AA' hairpin loops of other virus of the birnaviridae family can be identified by sequence comparison with the sequence of IBDV VP2 using the tools described above.

### 2. Conjugates and kit-of-parts of the invention

In a second aspect, the invention relates to a conjugate or kit-of-parts comprising
(i) a first component comprising the projection domain of the birnavirus VP2 wherein said first component lacks the AA' hairpin region and
(ii) a second component which is an immune co-stimulatory agent.

The term "conjugate", as used herein, refers to two or more compounds which are linked together so that the function of each compound is retained in the conjugate. As it will be described in detail below, the linkage between the first and second component of the conjugates can be covalent or non-covalent.

Those skilled in the art will observe that the first and second components of the conjugate may be provided forming such conjugate or, alternatively, they may be presented as separate formulations of each of the components, which may be combined for joint use as a combined preparation. Thus, the invention also relates to a kit-of-parts wherein the first and second components are individually formulated and packaged.

### 2.A. First component of the conjugate of the invention

The first component of the conjugate according to the present invention comprises the P domain of a birnavirus VP2 polypeptide, wherein said first component lacks the AA' hairpin region.

The elements of the first component of the conjugate of the invention correspond to the polypeptide of the invention and thus, they have been described above in detail.

### 2.B. Second component of the conjugate of the invention

The second component of the conjugates according to the invention is an immune co-stimulatory agent. Preferably, said co-stimulatory agent is not a region of said birnavirus VP2.

The term "Immune co-stimulatory polypeptide", as used herein, means any molecule that increases an individual's immune response against the first component.

### 2.B.I. TLR agonists

In a preferred embodiment, the Immune co-stimulatory agent is a TLR agonist.

The term "TLR agonist", as used herein, refers to a component which is capable of causing a signalling response through a TLR signalling pathway, either as a direct ligand or indirectly through generation of endogenous or exogenous ligand (Sabroe et al, JI 2003 p1630-5). Agonist ligands of TLR receptors are (i) natural ligands of the actual TLR receptor, or a functionally equivalent variant thereof which conserves the capacity to bind to the TLR receptor and induce co-stimulation signals thereon, or (ii) an agonist antibody against the TLR receptor, or a functionally equivalent variant thereof capable of specifically binding to the TLR receptor and, more particularly, to the extracellular domain of said receptor, and inducing some of the immune signals controlled by this receptor and associated proteins. The binding specificity can be for the human TLR receptor or for a TLR receptor homologous to the human one of a different species.

Toll-like receptors (or TLRs) are a family of type I transmembrane proteins forming part of the innate immune system. In vertebrates they also enable the adaptation of the immune system. TLRs together with interleukin receptors form a superfamily known as the Interleukin-1/toll-like receptor superfamily. All the members of this family have in common the domain called the Toll-IL-1 receptor (TIL) domain. It has been estimated that most mammals have between 10 and 15 types of TLRs. Thirteen types of TLRs have been identified up until now in human and mice (Du X, et al. 2000, Eur.Cytokine Netw. 11: 362-71; Chuang TH. et al., 2000. Eur. Cytokine Netw. 11: 372-378; Tabeta K, et al.; 2004, Proc. Natl. Acad. Sci. U.S.A. 101:3516-3521).

TLR agonists induce several immune responses depending on the cells in which the TLR is expressed as well as depending on the origin of TLR ligand. For example, in the case of microbial ligands, immune cells can produce cytokines which will cause inflammation. In the case of a viral factor, the cells can undergo apoptosis.

In one embodiment of the present invention, the TLR agonist is capable of causing a signalling response through TLR-1. Non-limiting examples of TLR-1 agonists include tri-acylated lipopeptides (LPs); phenol-soluble modulins; Mycobacterium tuberculosis LP; S-(2,3-bis(palmitoyloxy)-(2-RS)-propyl)-N-palmitoyl-(R)-Cys-(S)-Ser-(S)-Lys(4)-OH, trihydrochloride (Pam3Cys) LP which mimics the acetylated amino terminus of a bacterial lipoprotein and OspA LP from Borrelia burgdorfei.

In an alternative embodiment, the TLR agonist is capable of causing a signalling response through TLR-2. Non-limiting examples of TLR-2 agonists include, without limitation, one or more of a bacterial lipopeptide from *M. tuberculosis, B. burgdorferi, T. pallidum;* peptidoglycans from species including *Staphylococcus aureus;* lipoteichoic acids, mannuronic acids, Neisseria porins, bacterial fimbriae, Yersina virulence factors, CMV virions, measles haemagglutinin, and zymosan from yeast.

In an alternative embodiment, the TLR agonist is capable of causing a signalling response through TLR-3, such as double stranded RNA, or polyinosinic-polycytidylic acid (Poly I:C).

In an alternative embodiment, the TLR agonist is capable of causing a signalling response through TLR-4, such as the lipopolysaccharide (LPS) from gram-negative bacteria, or fragments thereof; heat shock protein (HSP) 10, 60, 65, 70, 75 or 90; surfactant Protein A, hyaluronan oligosaccharides, heparan sulphate fragments, fibronectin fragments, fibrinogen peptides and b-defensin-2. In one embodiment the TLR agonist is HSP 60, 70 or 90. In an alternative embodiment, the TLR agonist capable of causing a signalling response through TLR-4 is a non-toxic derivative of LPS such as monophosphoryl lipid A (MPL) as descrbed by Ribi et al (1986, Immunology and Immunopharmacology of bacterial endotoxins, Plenum Publ. Corp., NY, p407-419) and having the structure:

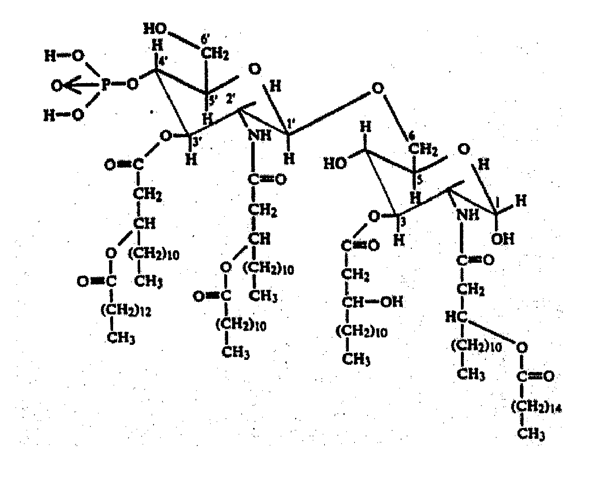

A further detoxified version of MPL results from the removal of the acyl chain from the 3- position of the disaccharide backbone, and is called 3-0-deacylated monophosphoryl lipid A (3D-MPL).

The non-toxic derivatives of LPS, or bacterial lipopolysaccharides, which may be used as TLR agonists in the present invention may be purified and processed from bacterial sources, or alternatively they may be synthetic. For example, purified monophosphoryl lipid A is described in Ribi et al 1986 (supra), and 3-0-Deacylated monophosphoryl or diphosphoryl lipid A derived from Salmonella sp. is described in GB 2220211 and US 4912094. Other purified and synthetic lipopolysaccharides have been described (US 6,005,099 and EP 0 729 473 B1; Hilgers et al., 1986, IntArch.Allergy.Immunol., 79(4):392-6; Hilgers et al., 1987, Immunology, 60(1):141-6; and EP 0 549 074 B1). Bacterial lipopolysaccharide adjuvants may be 3D-MPL and the f3(1-6) glucosamine disaccharides described in US 6,005,099 and EP 0 729 473 B1. Accordingly, other LPS derivatives that may be used as TLR agonists in the present invention are those immunostimulants that are similar in structure to that of LPS or MPL or 3D-MPL. In another aspect of the present invention the LPS derivatives may be an acylated monosaccharide, which is a sub-portion to the above structure of MPL. A disaccharide agonist may be a purified or synthetic lipid A of the following formula: 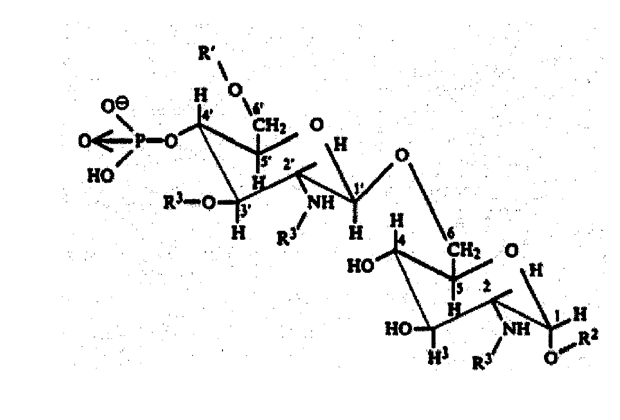 wherein R² may be H or PO₃H₂; R³ may be an acyl chain or 8-hydroxymyristoyl or a 3-acyloxyacyl residue having the formula: 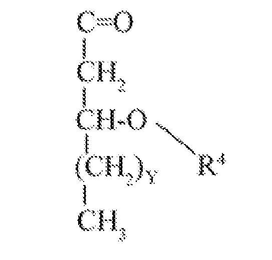 wherein R₄ is 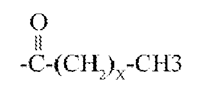 and X and Y have a value of 0 up to 20.

A yet further non-toxic derivative of LPS, which shares little structural homology with LPS and is purely synthetic is that described in WO 00/00462, the contents of which are fully incorporated herein by reference.

In a preferred embodiment, the immune co-stimulatory agent is a TLR ligand specific for TLR5 and, more in particular, flagelin or a functionally equivalent variant thereof. Suitable flagelins for use according to the present invention include the flagellin encoded by the fljB gene from Salmonella enterica serovar Typhymurium LT2 as well as any flagellin *Salmonella enterica* strains are known and publicly available on GenBank. Exemplary sequences include, but are not limited to, GenBank accession numbers AB108532, AY352264, AY353263, AY353262, AY353285, AY353284, AY353283, AY353282, AY353281, AY353280, AY353279, AY353278, AY353277, AY353276, AY353275, AY353274, AY353273, AY353272, AY353271, AY353271, AY353269, AY353268, AY353267, AY353266, AY353265, AY353307, AY353306, AY353305, and AY353303. In addition, other types of flagellins from Salmonella species can be used, so long as the flagellins can function as an adjuvant in a fusion polypeptide. For example, the phase 1 flagellin encoded by the fliC gene can be used. Exemplary fliC gene sequences are available on GenBank and include, but are not limited to, AY353368, AY353367, AY353366, AY353365, AY353364, AY353363, AY353362, AY353361, AY353360, AY353359, AY353358, AY353357, AY353356, AY353355, AY353354, AY353353, AY35352, AY353351, AY353350, and AY353349. Furthermore, flagellins from other Salmonella species, including flagellins having similar functions as those encoded by fliB and fliC genes, are well known and can be similarly be used as fusion polypeptides with an antigen to stimulate an immune response. Exemplary flagellin sequences are available on GenBank and include, but are not limited to, AY353261, AY353260, AY353259, AY353258, AE008826, D12510, AJ295701, AJ295700, AJ295699, AJ295698, AJ295697, AJ295696, AF045151, U17177, U17176, U17175, U17174, U17172, U17171, D13690, and D13689. Moreover, the flagellin component can include at least one member selected from the group consisting of an *E. coli* flagellin component, *a S. muenchen* flagellin component, a Yersinia flagellin component, a Campylobacter flagellin component, a *P. aeruginosa* flagellin component and a *L. monocytogenes* flagellin component.

In a preferred embodiment, the flagellin is the *Aliivibrio salmonicida* Flagellin A. In a still more preferred embodiment, the flagellin A component comprises the sequence

1 VNVNTNVAAM TAQRYLNNAT NDLNNSMERL SSGTKINSAK DDAAGLQISN RLTAQTRGLD

61 VAMRNANDGI SIAQTAEGAM NESTNILQRM RDLSLQAANG SNSNQDRSSI QEEVHALSDE

121 LNRIAETTSF GGRRLLNGQF GESAFQIGAN SGEAILIKLP SVRSDDDSMG GQRFVSENGK

181 TAEWNVETGK NDLKLEFTTK KGEAVSININ AKAGDDIEEL ATYINGQSDK ISASVGDEGK

241 LQLFVAEPSI EGELAVSGNL ASELGLAGGV GTKATVDKLD VTSVGDAQVS IGVIDSALAY
301 IDRERADLGA KQNRLGHSIN NLSNIQENVS ASNSRIKDTD FAKETTNMTK AQILQQSSTS
361 ILAQAKQLPN AALKLLQ (SEQ ID NO:7)

The term "functionally equivalent variant", as used herein, refers to any polypeptide which results from the modification by insertion, deletion or substitution of one or more amino acids of the flagelin molecules mentioned above and which maintains substantially the same TLR5 agonistic activity of flagelin.

In an alternative embodiment, the TLR agonist is capable of causing a signalling response through TLR-6 such as mycobacterial lipoprotein, di-acylated LP, and phenol-soluble modulin. Further TLR6 agonists are described in W02003043572.

In an alternative embodiment, the TLR agonist is capable of causing a signalling response through TLR-7 such as loxoribine, a guanosine analogue at positions N7 and C8, or an imidazoquinoline compound, or derivative thereof. In one embodiment, the TLR agonist is imiquimod. Further TLR7 agonists are described in W002085905.

In an alternative embodiment, the TLR agonist is capable of causing a signalling response through TLR-8 such as an imidazoquinoline molecule with anti-viral activity, for example resiquimod (R848); resiquimod is also capable of recognition by TLR-7. Other TLR-8 agonists which may be used include those described in W02004071459.

In an alternative embodiment, the TLR agonist is capable of causing a signalling response through TLR-9 such as s DNA containing unmethylated CpG nucleotides, in particular sequence contexts known as CpG motifs. CpG-containing oligonucleotides induce a predominantly Th1 response. Such oligonucleotides are well known and are described, for example, in WO 96/02555, WO 99/33488 and U.S. Patent Nos. 6,008,200 and 5,856,462. In one embodiment, CpG nucleotides are CpG oligonucleotides. In a preferred embodiment, the CpG oligonucleotide comprises the sequence 5'-TCGTCGTTTTGTCGTTTTGTCGTT-3' (SEQ ID NO:8). In a still more preferred embodiment, the CpG oligonucleotide contains phosphorothioate links.

In an alternative embodiment, the TLR agonist is capable of causing a signalling response through TLR-10. Alternatively, the TLR agonist is capable of causing a signalling response through any combination of two or more of the above TLRs.

"Immune cell" as used herein includes any cell that is involved in the generation, regulation or effect of the acquired or innate immune system. Immune cells include T cells such as CD4+ cells, CD8+ cells and various other T cell subsets, B cells, natural killer cells, macrophages, monocytes and dendritic cells, and neutrophils.

### 2.B.II. Fc receptor (FcR) binding polypeptides

The second component of the conjugates according to the invention may also be an Fc receptor (Fc) binding polypeptide. This allows the VP2 region of the conjugate to be presented to antigen-presenting cells (APC) once the conjugate binds the APC via interaction with Fc receptors on the surface of the APC (Lanza et al., 1993, Proc. Natl. Acad. Sci. USA 90:11683-11687).

The term "Fc receptor" or "FcR", as used herein, refers to a protein capable of specifically binding to a part of an antibody known as the Fc (Fragment, crystallizable). FcR is found on the surface of certain cells - including natural killer cells, macrophages, neutrophils, and mast cells - that contribute to the protective functions of the immune system. Its name is derived from its regionPreferred FcR binding polypeptide include the Fc segment of an immunoglobulin heavy chain constant region as well as a functionally equivalent variant thereof.

As used herein, the term "immunoglobulin heavy chain constant region" is used interchangeably with the term "Fc region" and is understood to mean the carboxyl-terminal portion of an immunoglobulin heavy chain constant region, or an analog or portion thereof capable of binding an Fc receptor.

As is known, each immunoglobulin heavy chain constant region comprises four or five domains. The domains are named sequentially as follows: CH1-hinge-CH2--CH3-CH4. CH4 is present in only IgY and is thus responsible for the higher molecular weight of this molecule with respect to IgG. The immunoglobulin heavy chain constant region useful in the practice of the invention preferably comprises an immunoglobulin hinge region, and preferably also includes a CH3 domain. The immunoglobulin heavy chain constant region most preferably comprises an immunoglobulin hinge region, a CH2 domain and a CH3 domain. As used herein, the term immunoglobulin "hinge region" is understood to mean an entire immunoglobulin hinge region or at least a portion of the immunoglobulin hinge region sufficient to form one or more disulfide bonds with a second immunoglobulin hinge region.

Different classes of immunoglobulin may be suitable for use in the present invention. The class or 'isotype' of an antibody is defined by its heavy chain, and in particular the sequence of the heavy chain region. In particular, immunoglobulins of the isotype IgG are most preferred, however antibodies of isotypes IgA, IgM, IgE, IgD, IgM and IgY may also have utility in various further embodiments of the present invention.

In certain embodiments, the immunoglobulin is derived from the same species that is to be treated with the immunoconjugate in order to minimise the occurrence of the Fc receptor binding peptide portion of the immunoconjugate being immunogenic. Thus, for treating diseases in birds, an Fc domain or an amino acid sequence derived from an aviar immunoglobulin would be preferred.

In a preferred embodiment, the FcR binding polypeptide is the Fc from IgY (Immunoglobulin Yolk). These define immunoglobulins which are extracted from the egg-yolk of fowl eggs and which correspond to the IgG in the serum of the chickens. These aviary immunoglobulins are structurally distinguished from the mammalian IgG primarily by their higher molecular weight resulting from a greater number of constant regions in the Fc fragment. The IgY Fc region comprises 4 domains known as Fc υ1, Fc υ2, Fc υ3 and Fc υ4.

In a preferred embodiment, the IgY Fc comprises regions Fc υ2, Fc υ3 and Fc υ4. In a still more preferred embodiment, the IgY Fc region comprises the sequence 
1 VCRVDPVPPV APEVQVLHPS SCTPSQSESV ELLCLVTGFS PASAEVEWLV

51 DGVGGLLVAS QSPAVRSGST YSLSSRVNVS GTDWREGKSY SCRVRHPATN

101 TVVEDHVKGC PDGAQSCSPI QLYAIPPSPG ELYISLDAKL RCLVVNLPSD

151 SSLSVTWTRE KSGNLRPDPM VLQEHFNGTY SASSAVPVST QDWLSGERFT

201 CTVQHEELPL PLSKSVYRNT GPTTPPLIYP FAPHPEELSL SRVTLSCLVR

251 GFRPRDIEIR WLRDHRAVPA TEFVTTAVLP EERTANGAGG DGDTFFVYSK

301 MSVETAKWNG GTVFACMAVH EALPMRFSQR TLQKQAGK (SEQ ID NO:9)
 wherein the underlined sequences correspond, respectively from the N- to the C-terminus, to the Fc υ2 and Fc υ4 domains.

In another preferred embodiment, the IgY Fc region used in the conjugates of the invention comprises regions Fc υ3 and Fc υ4. In a still more preferred embodiment, the IgY Fc region comprises the sequence 
1 GCPDGAQSCS PIQLYAIPPS PGELYISLDA KLRCLVVNLP SDSSLSVTWT

51 REKSGNLRPD PMVLQEHFNG TYSASSAVPV STQDWLSGER FTCTVQHEEL

101 PLPLSKSVYR NTGPTTPPLI YPFAPHPEEL SLSRVTLSCL VRGFRPRDIE

151 IRWLRDHRAV PATEFVTTAV LPEERTANGA GGDGDTFFVY SKMSVETAKW

201 NGGTVFACMA VHEALPMRFS QRTLQKQAGK (SEQ ID NO:10)
 which is formed by the IgY Fc υ3 and υ4 and further comprises the 8 amino acids from the Fc υ2 domain to preserve a C residue that could be involved in the protein folding through a disulfide bond together with the first C residue of the Fc υ3 domain.

Immunoglobulin heavy chain constant region domains have cross-homology among the immunoglobulin classes. Preferred immunoglobulin heavy chain constant regions include protein domains corresponding to one or more regions of the IgY selected from the group consisting of the CH1 region, the CH2 region, the CH3 region and the CH4 region or functional portions or derivatives thereof. The immunoglobulin heavy chain constant regions, however, preferably lack at least the CH1 domain.

In a more preferred embodiment, the immunoglobulin heavy chain constant region comprises, in an N to C terminal direction, an immunoglobulin hinge region, a CH2 domain and a CH3 domain all of which are based on sequences from an IgG molecule. The choice of appropriate immunoglobulin heavy chain constant regions is discussed in detail in U.S. Pat. Nos. 5,541,087, and 5,726,044. The choice of particular immunoglobulin heavy chain constant region sequences from certain immunoglobulin classes and subclasses to achieve a particular result is considered to be within the level of skill in the art.

It may be useful, in some circumstances, to modify the immunoglobulin heavy chain constant region, for example, by mutation, deletion or other changes mediated by genetic engineering or other approaches, so that certain activities, such as complement fixation or stimulation of antibody-dependent cell-mediated cytotoxicity (ADCC) are reduced or eliminated. However, it is considered necessary that the immunoglobulin heavy chain constant region's ability to bind an Fc receptor is maintained. In the practice of this invention, the immunoglobulin heavy chain constant region component of the conjugates comprising Fc according to the invention is non-immunogenic or is weakly immunogenic in the intended recipient. The Fc region is considered non- or weakly immunogenic if the immunoglobulin heavy chain constant region fails to generate a detectable antibody response directed against the immunoglobulin heavy chain constant region. Accordingly, the immunoglobulin heavy chain constant region should be derived from immunoglobulins present, or based on amino acid sequences corresponding to immunoglobulins present in the same species as the intended recipient of the fusion protein. In other words, human immunoglobulin constant heavy region sequences should be used when the Fc fusion construct (the Fc-antigen and/or the Fc-adjuvant fusion protein) is to be administered to a human. Nucleotide and amino acid sequences of human Fc IgG are disclosed, for example, in Ellison et al. (1982) NUCLEIC ACIDS RES. 10:4071-4079. Likewise, murine Fc sequences should be used when the Fc fusion is to be administered to mice. Nucleotide and amino acid sequences of murine Fc IgG2a are disclosed, for example, in Bourgois et al. (1974) EUR J. BIOCHEM. 43:423-435. The same logic would be applied if the Fc fusion proteins were to be administered to other animals including pets, for example, cats and dogs, and farm animals, for example, cows and horses.

The term "functionally equivalent variant", when referring to the immunoglobulin heavy chain constant region, as used herein, refers to any polypeptide resulting from the deletion, insertion or replacement of one or more residues in the immunoglobulin heavy chain constant region and which substantially preserves its FcR binding properties. Functionally equivalent variants are those showing a degree of identity with respect to the the immunoglobulin heavy chain constant region higher than at least 25%, at least 40%, at least 60%, at least 70%, at least 80%, at least 90%, at least 95%, at least 96%, at least 97%, at least 98% or at least 99%. As used herein, "% sequence identity" is determined by comparing two optimally aligned sequences over a comparison window, where the fragment of the polypeptide or polynucleotide sequence in the comparison window may comprise additions or deletions (e.g., gaps or overhangs) as compared to the reference sequence (which does not comprise additions or deletions) for optimal alignment of the two sequences. The percentage is calculated by determining the number of positions at which the identical amino acid residue or nucleic acid residue occurs in both sequences to yield the number of matched positions, dividing the number of matched positions by the total number of positions in the comparison window and multiplying the result by 100 to provide the percentage of sequence identity. Algorithms to align sequences are known in the art. Exemplary algorithms include, but are not limited to, the local homology algorithm of Smith and Waterman (Add APL Math, 2:482, 1981); the homology alignment algorithm of Needleman and Wunsch (J Mol Biol 1970;48:443); the search for similarity method of Pearson and Lipman (Proc Natl Acad Sci USA 1988:85:2444); and computerized implementations of these algorithms (GAP, BESTFIT, BLAST, PASTA, and TFASTA in the Wisconsin Genetics Software Package, Genetics Computer Group (GCG), 575 Science Dr., Madison, Wis.). In one embodiment, two sequences may be aligned using the "Blast 2 Sequences" tool at the NCBI website at default settings (Tatusova and Madden. FEMS Microbiol Lett 1999;174:247-250). Alternatively, amino acid sequences or nucleic acids sequences may be aligned by human inspection.

The capacity of the functionally equivalent variant of the immunoglobulin heavy chain constant region for specifically binding to FcR is determined using conventional methods known by the person skilled in the art. The term "specifically binds" or "binding specificity" refers to the ability of the FcR binding peptide to bind to a target epitope present on the Fc receptor or the homologue thereof with a greater affinity than it binds to a non-target epitope. In certain embodiments, specific binding refers to binding of the FcR binding peptide to a target epitope present on the FcR with an affinity which is at least 10, 50, 100, 250, 500, or 1000 times greater than the affinity for a non-target epitope. For example, by way of illustration, the capacity of the variant to bind FcR can be determined using co-immunoprecipitation experiments, in which the protein of interest is isolated with a specific antibody and the molecules which interact with the protein (e.g. FcR) are subsequently identified by means of a western blot. Alternatively, binding affinity may be determined by different means such as, without limitation, affinity ELISA assay or a BIAcore assay or by a kinetic method.

In the particular case when a variant of the Fc region is that of a IgY, the determination of whether the variant is a functionally equivalent variant can be determined as described by Pürzel et al. (J.Immunol., 2009, 183: 4554-4559) by determining the ability of the variant to promote expression of a reporter gene in a cell expressing a fusion protein of CHIR-AB1-CD3ξ comprising the extracytoplasmic Ig domain of the chicken Ig-like receptor CHIR-AB1 and the cytoplasmic domain of murine CD3ξ and a reporter gene under the control of a CD3ξ-sensitive promoter.

Assays for determining the capacity of the functionally equivalent variants of the Fc region to promote presentation of the peptide attached thereto on monocytes can be determined, e.g. as described by Liu et al. (J.Clin.Invest., 1996, 98:2001-2007) by measuring the ability of the conjugate comprising the Fc variant to promote proliferation of T cells in the presence of monocytes previously exposed to said conjugate.

### 2.B.III. CD40 agonists

In another embodiment, the co-stimulatory molecule is a CD40 agonist. The term "CD40 agonist", as used herein, refers to a compound that binds to the CD40 receptor and triggers signaling in a manner similar to the endogenous CD40 ligand. Assays adequate for determining whether a compound is capable of acting as a CD40 ligand are those based on the detection of either the increase in the expression of more CD40 and TNF receptors in macrophages or the activation of B cells and their transformation into plasma cells. The activation of B-cells in response to a CD40 ligand can be assayed by measuring the increase in Inositol 1,4,5-Trisphosphate levels or the activation of tyrosine kinases as described by Uckun et al. (J Biol Chem 1991;26:17478-17485). Alternatively, the determination of whether a compound is a CD40 agonist can be carried out for example, in macrophages that expressed CD40 on the membrane. In said macrophages, when a CD40-agonist-bearing-Tcell interacts with the macrophage, the macrophage expresses more CD40 and TNF receptors on its surface which helps to increase the level of activation. The increase in activation results in the introduction of potent microbicidal substances in the macrophage, including reactive oxygen species and nitric oxide.

Suitable CD40 agonists for use in the present invention include, without limitation, soluble CD40 Ligand (CD40L), a functionally equivalent variant of the CD40 ligand, CD40L fragments (such as the ones described in WO2009141335), conjugates and derivatives thereof such as oligomeric CD40L polypeptides, e.g., trimeric CD40L polypeptides, the C4BP Core protein (the C-terminal domain of the alpha chain of C4BP) as described in W005051414 and a CD40 agonistic antibody.

In a preferred embodiment, the CD40 agonist is a CD40 agonistic antibody (such as those described in US2008286289, US2007292439, US2005136055).

### 2.B.IV. Other immune co-stimulatory molecules

Exemplary immune co-stimulatory molecules (polypeptides) useful in accordance with the invention include, without limitation, LIGHT, CD80 (B7-1), CD86 (B7-2), ICOS, ICOSL (including B7h, B7-H2, B7RP-1, GL-50 and LICOS), CD94 (KP43), CD40L (CD154), ICAM-1 (CD54), ICAM-2, ICAM-3, SLAM (CD150), HAS (CD24), 4-1BB (CDw137), 4-1BBL (CDw137L), OX40L, CD28, CD40 (BP50), CD25 (IL-2R alpha), Lymphotoxin (LT alpha or LT beta), TNF, Fas-L, GITR (activation-inducible TNRF), GITR Ligand, CD11a (alpha L integrin), CD11b (alpha M integrin), L-selectin (CD62L), CD69 (very early activation antigen), CD70 (CD27L), PD-L1, PD-L2, B7-H3, B7-H4, OX40L, 4-1BBL, CD27L, CD30L, LIGHT, BAFF, and APRIL. See e.g., Watts and DeBenedette, 1999, Curr. Opin. Immunol., 11:286-93.

### 2. C. Polypeptide tags

In a preferred embodiment, the conjugate of the invention further comprises a "tag". The term "tag", as used herein, relates to any amino acid sequence for which specific binding molecules are available, thus allowing the detection/purification of any polypeptide carrying said tag. The tag is generally placed at the amino- or the carboxyl-terminus of the polypeptide. The presence of such tag allows the adapter molecule to be detected using an antibody against the tag polypeptide. Also, the provision of the tag enables the adapter polypeptide to be readily purified by affinity purification using an anti-tag antibody or another type of affinity reagent that binds to the epitope tag.

Various tag polypeptides and their respective antibodies are well known in the art. Examples include poly-histidine (poly-his) or poly-histidine-glycine (poly-his-gly) tags; the flu HA tag polypeptide and its antibody 12CA5 (Field et al., Mol Cell Biol, 1988;8:2159-2165); the c-myc tag and the 8F9, 3C7, 6E10, G4, B7 and 9E10 antibodies thereto *(*Evan et al., Molecular and Cellular Biology 1985;5:3610-3616); the Herpes Simplex virus glycoprotein D (gD) tag and its antibody (Paborsky et al., Protein Engineering 1990;3:547-553). Other tag polypeptides include the Flag-peptide (Hopp et al., BioTechnology 1988;6:1204-1210); the KT3 epitope peptide [Martin et al., Science 1993;255:192-194); tubulin epitope peptide *(*Skinner et al., J Biol Chem 1991;266:15163-15166); and the T7 gene 10 protein peptide tag (Lutz-Freyermuth et al., Proc Natl Acad Sci USA 1990,;87:6393-6397). In a preferred embodiment, the purification tag is a polyhistidine tag. In a still more preferred embodiment, the purification tag is an hexahistidine tag.

### 3. Structure of conjugates

### 3.A. Direct coupling

The conjugates according to the invention may be formed by a direct binding of the first and second components, i.e. by means of a specific direct interaction between the component comprising the projection domain and the immune co-stimulatory agent. The first and second components may be directly coupled or may be linked via a polypeptide linker.

The skilled person will appreciate that the conjugates may result from the chemical cross-linking of the first and second components. However, in the particular case that the co-stimulatory agent is a polypeptide, it is preferred that the first and second components are connected as a result of "genetic cross-linking", i.e. wherein the first and second components form a single polypeptide.

The conjugates may include a linker such as a peptide linker between the first and second components. The linker length and composition may be chosen to enhance the activity of either or both functional ends of the conjugate (e.g., co-stimulatory polypeptide/antigen.infectious agent or binding pair member). The linker is generally from about 3 to about 15 amino acids long, more preferably about 5 to about 10 amino acids long, however, longer or shorter linkers may be used or the linker may be dispensed with entirely. Flexible linkers (e.g. (Gly₄Ser₃) such as have been used to connect heavy and light chains of a single chain antibody may be used in this regard. See Huston, et al. (1988) Proc. Nat. Acad. Sci. USA, 85:5879-5883; U.S. Pat. Nos. 5,091,513, 5,132,405, 4,956,778; 5,258,498, and 5,482,858. Other linkers are FENDAQAPKS or LQNDAQAPKS. One or more domains of and immunoglobulin Fc region (e.g. CH1, CH2 and/or CH3) also may be used as a linker. Chemical linkers also may be used.

Conjugates forming a fusion protein may have a variety of configurations. In one embodiment, the C-terminus of the component comprising the projection domain of the birnavirus VP2 is linked to the N-terminus of the immune co-stimulatory agent. Alternatively, the C-terminus of the immune co-stimulatory agent is linked to the N-terminus of the component comprising the projection domain of the birnavirus. Furthermore, it is contemplated that the fusion proteins may comprise a plurality of one or more of the first component or one or more of the second component.

In a preferred embodiment, the conjugate according to the invention is the conjugate known as "FlagA_HT_VP2-PD" which comprises, in the N- to C- orientation, a second component comprising the *Aliivibrio salmonicida* Flagellin A, an hexahistidine tag and a first component comprising the projection domain of the birnavirus VP2 wherein said birnavirus VP2 projection domain lacks the AA' hairpin region.

In a still more preferred embodiment, the conjugate according to the invention comprises the sequence 
1 ADPGVNVNTN VAAMTAQRYL NNATNDLNNS MERLSSGTKI NSAKDDAAGL

51 QISNRLTAQT RGLDVAMRNA NDGISIAQTA EGAMNESTNI LQRMRDLSLQ

101 AANGSNSNQD RSSIQEEVHA LSDELNRIAE TTSFGGRRLL NGQFGESAFQ

151 IGANSGEAIL IKLPSVRSDD DSMGGQRFVS ENGKTAEWNV ETGKNDLKLE

201 FTTKKGEAVS ININAKAGDD IEELATYING QSDKISASVG DEGKLQLFVA

251 EPSIEGELAV SGNLASELGL AGGVGTKATV DKLDVTSVGD AQVSIGVIDS

301 ALAYIDRERA DLGAKQNRLG HSINNLSNIQ ENVSASNSRI KDTDFAKETT

351 NMTKAQILQQ SSTSILAQAK QLPNAALKLL QLEACASGRL VPRGSPGSGY

401 IPEAPRDGQA YVRKDGEWVL LSTFLGHHHH HHDYDIPTTE NLYFQGAMGS

451 DRPRVYTITA ADDYQFSSQY QPGGVTITLF SANIDAITSL SVGGELVFRT

501 SVHGLVLGAT IYLIGFDGTT VITRAVAANN GLTTGTDNLM PFNLVIPTNE
 551 ITQPITSIKL EIVTSKSGGQ AGDQMSWSAR GSLAVTIH (SEQ ID NO:11) wherein the underlined region corresponds to the *Aliivibrio salmonicida* Flagellin A, the region in italics corresponds to the hexahistidine tag and the double underlined region corresponds to the VP2 P domain. The rest of the sequences derive from the expression vector.

In a preferred embodiment, the conjugate according to the invention comprises, in the N- to C- orientation, the *Gallus gallus* IgY Fc (second component), an hexahistidine tag and the projection domain of the birnavirus VP2 wherein said birnavirus VP2 projection domain lacks the AA' hairpin region (first component).

In another embodiment, the conjugate according to the invention comprises is the IgY Fc υ2-4_HT_P which comprises the *Gallus gallus* IgY Fc υ2-4 region, an hexahistidine tag and the IBDV VP2 P domain and which has the sequence 
1       ADPGVCRVDP  VPPVAPEVQV  LHPSSCTPSQ  SESVELLCLV  TGFSPASAEV

51      EWLVDGVGGL  LVASQSPAVR  SGSTYSLSSR  VNVSGTDWRE  GKSYSCRVRH

101     PATNTVVEDH  VKGCPDGAQS  CSPIQLYAIP  PSPGELYISL  DAKLRCLVVN

151     LPSDSSLSVT  WTREKSGNLR  PDPMVLQEHF  NGTYSASSAV  PVSTQDWLSG

201     ERFTCTVQHE  ELPLPLSKSV  YRNTGPTTPP  LIYPFAPHPE  ELSLSRVTLS

251     CLVRGFRPRD  IEIRWLRDHR  AVPATEFVTT  AVLPEERTAN  GAGGDGDTFF

301     VYSKMSVETA  KWNGGTVFAC  MAVHEALPMR  FSQRTLQKQA  GKLEACASGR

351     LVPRGSPGSG  YIPEAPRDGQ  AYVRKDGEWV  LLSTFLGHHH  HHHDYDIPTT

401     ENLYFQGAMG  SDRPRVYTIT  AADDYQFSSQ  YQPGGVTITL  FSANIDAITS

451     LSVGGELVFR  TSVHGLVLGA  TIYLIGFDGT  TVITRAVAAN  NGLTTGTDNL

501     MPFNLVIPTN  EITQPITSIK  LEIVTSKSGG  QAGDQMSWSA  RGSLAVTIH  (SEQ ID
NO:12)
 wherein the underlined region corresponds to the *Gallus gallus* IgY Fc υ2-4, the region in italics corresponds to the hexahistidine tag and the double underlined region corresponds to the VP2 P domain. The rest of the sequences derive from the expression vector.

In a still more preferred embodiment, the conjugate according to the invention is the conjugate known as IgY Fc υ3-4_HT_P which comprises the *Gallus gallus* IgY Fc υ3-4 region, an hexahistidine tag and the IBDV VP2 P domain and which comprises the sequence 
1       ADPGCPDGAQ  SCSPIQLYAI  PPSPGELYIS  LDAKLRCLVV  NLPSDSSLS

51      VTWTREKSGN  LRPDPMVLQE  HFNGTYSASS  AVPVSTQDWL  SGERFTCTVQ

101     HEELPLPLSK  SVYRNTGPTT  PPLIYPFAPH  PEELSLSRVT  LSCLVRGFRP

151     RDIEIRWLRD  HRAVPATEFV  TTAVLPEERT  ANGAGGDGDT  FFVYSKMSVE

201     TAKWNGGTVF  ACMAVHEALP  MRFSQRTLQK  QAGKLEACAS  GLVPRGSPGS

251     GYIPEAPRDG  QAYVRKDGEW  VLLSTFLGHH  HHHHDYDIPT  TENLYFQGAM

301     GSDRPRVYTI  TAADDYQFSS  QYQPGGVTIT  LFSANIDAIT  SLSVGGELVF

351     RTSVHGLVLG  ATIYLIGFDG  TTVITRAVAA  NNGLTTGTDN  LMPFNLVIPT

401     NEITQPITSI  KLEIVTSKSG  GQAGDQMSWS  ARGSLAVTIH  (SEQ ID NO:13)
 wherein the underlined region corresponds to the *Gallus gallus* IgY Fc υ2-4, the region in italics corresponds to the hexahistidine tag and the double underlined region corresponds to the VP2 P domain. The rest of the sequences results from the expression vector.

Moreover, in the particular case when the immune co-stimulatory agent is an FcR binding polypeptide, two or more Fc-antigen fusion proteins may be associated together either non-covalently or covalently, for example, through one or more disulfide bonds, to produce dimeric or multimeric compositions.

### 3.B. Coupling through a binding pair

Alternatively, the first and second components of the conjugates according to the invention can be bound indirectly, i.e. by means of using a "binding pair", wherein a first member of a binding pair is coupled to the first component and a second member of a binding pair coupled to the immune co-stimulatory agent. Thus, by contacting both fusion molecules, a complex is formed via the interaction of the first and second members of the binding pair.

### 3.B.I. Binding pairs

As used herein, the term "first member of a binding pair" and "second member of a binding pair" relates to a pair of molecules which can interact specifically and bind to each other. The first and/or second members of the binding pair can be of a peptide (protein) or non-peptide nature.

As used herein, the expression "which can interact specifically" or "specific interaction" describes the interaction between a first species and a second species characterized in that the nature of binding is such that an antibody, a receptor or a nucleic acid binding protein binds to its corresponding binding pair but does not substantially bind to other species. Likewise, the term "binding" or "binding to", as used herein, means the physical association between a first species and a second species, for example, the binding of a ligand by a receptor, the binding of an antigen by an antibody, the binding of a nucleic acid by a nucleic acid binding protein, etc.

The term "binding pair" does not involve any particular size or any other technical structural characteristic other than that said binding pair can interact and bind to the other member of the binding pair resulting in a conjugate wherein the first and second components are bound to each other by means of the specific interaction between the first and second member of a binding pair. By way of illustration, suitable binding pairs are e.g. biotin-avidin, epitope-antibody or functional fragment thereof (e.g. a Fab or F(ab')2 fragment, an scFv (single chain antibody), a chimeric antibody, etc.), lectin-carbohydrate, etc., in which each of molecules are the members of said specific binding pair, each of them containing the corresponding binding domains or regions of interaction.

The binding pair includes any type of interaction of immune type such as antigen/antibody, antigen/antibody fragment, hapten/anti-hapten as well as interactions of non-immune type such as avidin/biotin, avidin/biotinylated molecules, folic acid/folate-binding protein, hormone/hormone receptor, lectin/carbohydrate, lectin/molecule modified with carbohydrates, enzyme/enzyme substrate, enzyme/enzyme inhibitor, protein A/antibody, protein G/antibody, complementary nucleic acids (including sequences of DNA, RNA and peptide nucleic acids (PNA)), polynucleotide/polynucleotide-binding protein and the like.

As used in the present invention, the expression "specific binding" refers to the capacity of a first molecule to bind specifically to a second molecule by means of the existence of complementarity between the three-dimensional structures of the two molecules with a substantially higher affinity for non-specific binding such that the binding between said first and second molecule preferably takes place before the binding of any of said molecules with respect to the other molecules present in the reaction mixture. It is understood that there is high affinity in the binding of two molecules when the complex resulting from said binding has a dissociation constant (KD) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M or less than 10⁻¹⁵ M.

The terms "bond" and "binding" are used indistinctly to refer to an interaction between two or more entities. In those cases in which two entities are bound to one another, they can be directly bound (for example, by means of covalent bonds, ionic forces, hydrogen bonds, electrostatic interactions, Van der Waals forces or a combination of the above) or they can be indirectly bound, for example, by means of a linker.

In a preferred embodiment, the first member of a binding pair is a biotin-binding molecule. More preferably, the biotin-binding molecule is avidin, streptavidin (SA) or SA or avidin fragments which retain substantial binding activity for biotin, such as at least 50 percent or more of the binding affinity of native SA. As used herein, the term "avidin" refers to a glycoprotein found in egg whites and in tissues of birds, reptiles and amphibians protein and which has the capacity to bind to biotin with high affinity as well as any expressed or engineered form of the avidin biotin-binding molecule, such as streptavidin, neutravidin and the like. The term avidin includes both avidin found naturally in the eggs of *Gallus gallus* (NCBI accession numbers NM_205320.1 / GL45384353en) as well as the orthologues of said protein in other species. Streptavidin, corresponding to the protein from *Streptomyces avidinii* (accession number CAA00084.1 in GenBank), as well as the orthologues, homologues and fragments of streptavidin defined in the same manner as avidin. Streptavidin comprises 4 subunits each of which contains a binding site for biotin. Streptavidin (SA) or avidin fragments which retain substantial binding activity for biotin, such as at least 50 percent or more of the binding affinity of native SA or avidin, respectively, may also be used. Preferably, the affinity of the avidin variant for biotin is of at least 10¹⁵ M⁻¹, 10¹⁴ M⁻¹, 10¹³ M⁻¹, 10¹² M⁻¹, 10¹⁰ M⁻¹ or 10⁹ M⁻¹_{.}

Avidin and estreptavidin variants suitable for use in the present invention include, without limitation:
- "Core streptavidin" ("CSA"), which is a truncated version of the full-length streptavidin polypeptide which may include streptavidin residues 13-138, 14-138, 13-139 and 14-139. See, e.g., Pahler et al., (J. Biol. Chem. 1987, 262: 13933-37).
- Truncated forms of streptavidin and avidin that retain strong binding to biotin (See, e.g. Sano et al., (J Biol Chem., 1995, 270: 28204-09) (describing core streptavidin variants 16-133 and 14-138) (U.S. Pat. No. 6,022,951).
- Mutants of streptavidin and core forms of streptavidin which retain substantial biotin binding activity or increased biotin binding activity. See Chilcoti et al., Proc Natl. Acad. Sci. USA. Feb. 28, 1995;92(5):1754-8; Reznik et al., Nat Biotechnol. August 1996;14(8):1007-1 1.
- Mutants with reduced immunogenicity, such as mutants mutated by site-directed mutagenesis to remove potential T cell epitopes or lymphocyte epitopes,. See Meyer et al., Protein Sci. 2001 10: 491-503.
- Mutants of avidin and core forms of avidin which retain substantial biotin binding activity or increased biotin binding activity also may be used. See Hiller et al., J. Biochem. (1991) 278: 573-85; Livnah et al. Proc. Natl. Acad. Sci. USA (90: 5076-80 (1993).
- Variants resulting from the chemical modification of avidin such as those resulting from the complete or partial modification of glycosylation and fragments thereof as well as the completely deglycosylated avidin variant known as neutravidin.
- Avidin mutants as described in WO05047317A1
- Avidin-like proteins as described in WO06045891,
- Recombinant avidin as described in W00198349,
- Avidin variants as described in WO0027814,
- Monomeric streptavidin as described in W006084388,
- Modified streptavidin dimers such as those described in WO06058226,
- The protein with biotin binding capacity as described in WO04018509,
- Streptavidin having a higher affinity for biotin as described in WO9840396,
- The modified streptavidin and avidin molecules as described in WO9640761,
- The streptavidin mutants as described in W09711183,
- The streptavidin with modified affinity as described in WO9624606.

For convenience, in the instant description, the terms "avidin" and "streptavidin" as used herein are intended to encompass biotin-binding fragments, mutants and core forms of these binding pair members. Different avidin variants are commercially available, such as Extravidin (Sigma-Aldrich), NeutrAvidin (Thermo Scientific), NeutrAvidin (Invitrogen) and NeutraLite (Belovo). Moreover, the nucleic acid sequences encoding streptavidin and avidin and the streptavidin and avidin amino acid sequences can be found, for example, in GenBank Accession Nos. X65082; X03591; NM --205320; X05343; Z21611; and Z21554.

In a preferred embodiment, the first member of the binding pair comprises a Monomeric avidin. In a still more preferred embodiment, the monomeric avidin results from avidin comprising the N54A and W110K mutations as described by Laitinen OH et al. J. Biol. Chem. 2003, 278:4010-4014). In a still more preferred embodiment, the Monomeric avidin used in the present invention comprises the sequence 
1   ARKCSLTGKW  TNDLGSNMTI  GAVNSRGEFT  GTYITAVTAT  SNEIKESPLH

51   GTQATINKRT  QPTFGFTVNW  KFSESTTVFT  GQCFIDRNGK  EVLKTMWLLR

101   SSVNDIGDDK  KATRVGINIF  TRLRTQKE (SEQ ID NO:14)
 wherein underlined residues correspond to the mutated amino acids (N54A and W110K) that allow express the protein in a monomeric state. (Laitinen OH et al. J. Biol. Chem. 2003, 278:4010-4014)

In accordance with one embodiment, the polypeptide comprising the P domain of the birnavirus VP2 protein is bound via its C-terminus to the N-terminus of the binding pair member. For example, the polypeptide comprising the P domain of the birnavirus VP2 can be bound via its C-terminus to the N-terminus of core streptavidin (CSA). Thus the invention includes VP2 PD-CSA fusion proteins, where the projection domain of the birnavirus VP2 lacking the AA' hairpin region is bound via its C-terminal to the N-terminal of CSA.

In accordance with another embodiment, the immune co-stimulatory polypeptide is bound via its N-terminus to the C-terminus of the binding pair member. For example, an immune co-stimulatory polypeptide can be bound via its N-terminus to the C-terminus of CSA. For example, the invention includes CSA-VP2 PD fusion proteins, wherein the CSA moiety is bound via its C-terminal to the N-terminal of the polypeptide comprising the P domain of the birnavirus VP2 protein.

In a preferred embodiment the biotin binding molecule is a mutant version of the chicken avidin (mAV) polypeptide designed to form complexes with biotinylated macromo lecules.

As used herein "biotin" includes biotin-containing moieties that are able to bind to surfaces, such as cell surfaces (including tumor cell surfaces), such as NHS-biotin and EZ-Link.TM. Sulfo-NHS-LC-Biotin (Pierce). Such protein reactive forms of biotin are available commercially.

The interaction between biotin and its binding partner, avidin or streptavidin, offers several advantages in the context of the present invention. For example, biotin has an extremely high affinity for both streptavidin (10¹³ M⁻¹) and avidin (10¹⁵ M⁻¹). Additionally, both streptavidin and avidin are tetrameric polypeptides that each bind four molecules of biotin. Immune co-stimulatory moieties comprising streptavidin or avidin therefore have a tendency to form tetramers and higher structures. As a result, they can cross-link their corresponding immune cell receptors for more potent signal transduction, such as through aggregation of receptors.

In order for the complexes between the first component of the conjugate modified with a first member of a binding pair and the co-stimulatory molecule, such co-stimulatory molecule is coupled to a second member of binding pair.

In a preferred embodiment, the conjugate according to the invention comprises a fusion protein comprising the IBDV VP2 P domain and monomeric avidin and biotinylated CpG oligonucleotides. In a still more preferred embodiment, the fusion protein comprising the IBDV VP2 P domain and monomeric avidin comprises the sequence 
1 ADPGSDRPRV YTITAADDYQ FSSQYQPGGV TITLFSANID AITSLSVGGE

51 LVFRTSVHGL VLGATIYLIG FDGTTVITRA VAANNGLTTG TDNLMPFNLV

101 IPTNEITQPI TSIKLEIVTS KSGGQAGDQM SWSARGSLAV TIHGGLEACA

151 SGRLVPRGSP GSGYIPEAPR DGQAYVRKDG EWVLLSTFLG HHHHHHDYDI

201 PTTENLYFQG AMARKCSLTG KWTNDLGSNM TIGAVNSRGE FTGTYITAVT

251 ATSNEIKESP LHGTQATINK RTQPTFGFTV NWKFSESTTV FTGQCFIDRN

301 GKEVLKTMWL LRSSVNDIGD DKKATRVGIN IFTRLRTQKE (SEQ ID NO:15)
 wherein the underlined region corresponds to the VP2 P domain, the region in italics corresponds to the hexahistidine tag and the double underlined region corresponds to the mAV. The rest of the sequences derive from the expression vector.

In a still more preferred embodiment, the biotinylated CpG oligonucleotide comprises the sequence 5'-TCGTCGTTTTGTCGTTTTGTCGTT-3' (SEQ ID NO:8)..

### 4. Methods for preparing the conjugates according to the invention

### 4.A. Conjugates wherein components are directly connected

A conjugate according to the present invention can be made by methods well known in the art. For example, the first and second components of the conjugate can be covalently bound to each other or conjugated to each other directly or through a linker.

The conjugate of the invention can be obtained using any method known for a person skilled in the art. It is thus possible to obtain the polypeptide comprising the projection domain of the birnavirus VP2 lacking the AA' hairpin region by any standard method. For example, the polypeptide can be obtained from cDNA by means of expression in a heterologous organism such as, for example, *Escherichia coli, Saccharomyces cerevisiae, Pichia pastoris.*

Once a sufficient amount of the purified polypeptide is available, the latter must be conjugated to the first member of the binding pair. The conjugation of said first member of the binding pair to the polypeptide molecule can be carried out in different ways. One possibility is the direct conjugation of a functional group to polypeptide comprising the P domain of the birnavirus VP2 in a position which does not interfere with the activity of said component. As understood in the present invention, functional groups refer to a group of specific atoms in a molecule which are responsible for a characteristic chemical reaction of said molecule. Examples of functional groups include, without limitation, hydroxy, aldehyde, alkyl, alkenyl, alkynyl, amide, carboxamide, primary, secondary, tertiary and quaternary amines, aminoxy, azide, azo (diimide), benzyl, carbonate, ester, ether, glyoxylyl, haloalkyl, haloformyl, imine, imide, ketone, maleimide, isocyanide, isocyanate, carbonyl, nitrate, nitrite, nitro, nitroso, peroxide, phenyl, phosphine, phosphate, phosphono, pyridyl, sulfide, sulfonyl, sulfinyl, thioester, thiol and oxidized 3,4-dihydroxyphenylalanine (DOPA) groups. Examples of said groups are maleimide or glyoxylyl groups, which react specifically with thiol groups in the Apo A molecule and oxidized 3,4-dihydroxyphenylalanine (DOPA) groups which react with primary amino groups in the polypeptide forming component (i).

Another possibility is to conjugate the first member of the binding pair to the polypeptide comprising the P domain of the birnavirus VP2e by means of the use of homo- or hetero- bifunctional groups. The bifunctional group can first be conjugated to the therapeutically active compound and, then, conjugated to the polypeptide or, alternatively, it is possible to conjugate the bifunctional group to the polypeptide and, then, conjugate the latter to component (ii). Illustrative examples of this type of conjugates include the conjugates known as ketone-oxime (described in US20050255042) in which the first component of the conjugate comprises an aminoxy group which is bound to a ketone group present in a heterobifunctional group which, in turn, is bound to an amino group in the second component of the conjugate.

In another embodiment, the agent used to conjugate components (i) and (ii) of the conjugates of the invention can be photolytically, chemically, thermically or enzymatically processed. In particular, the use of linking agents which can be hydrolyzed by enzymes that are in the target cell, such that the therapeutically active compound is only released into the cell, is of interest. Examples of linking agent types that can be intracellularly processed have been described in WO04054622, WO06107617, WO07046893 and W007112193.

In a preferred embodiment, wherein the first member of a binding pair is a compound of a peptide nature (e.g., streptavidin), including both oligopeptides, peptides and proteins, it is possible to chemically modify a polypeptide chain using widely known methods to the person skilled in the art so that the protein can be covalently coupled to a second polypeptide. Thus, suitable methods for the covalent coupling of two polypeptides include methods based on the conjugation through the thiol groups present in the cysteine moieties, methods based on the conjugation through the primary amino groups present in the lysine moieties (US6809186), methods based on the conjugation through the N- and C-terminal moieties can be used. Reagents suitable for the modification of polypeptides to allow their coupling to other compounds include: glutaraldehyde (allows binding compounds to the N-terminal end of polypeptides), carbodiimide (allows binding the compound to the C-terminal end of a polypeptide), succinimide esters (for example MBS, SMCC) which allow activating the N-terminal end and cysteine moieties, benzidine (BDB), which allows activating tyrosine moieties, and periodate, which allows activating carbohydrate moieties in those proteins which are glycosylated.

In accordance with one embodiment, the first and second component form a fusion protein. Fusion proteins can be made by any of a number of different methods known in the art. For example, one or more of the component polypeptides of the fusion proteins can be chemically synthesized or can be generated using well known recombinant nucleic acid technology. (As used herein, "nucleic acid" refers to RNA or DNA.) Nucleic acid sequences useful in the present invention can be obtained using, for example, the polymerase chain reaction (PCR). Various PCR methods are described, for example, in PCR Primer: A Laboratory Manual, Dieffenbach 7 Dveksler, Eds., Cold Spring Harbor Laboratory Press, 1995.

In the particular case in which the first and second components of the conjugate according to the invention form a single polypeptide chain, it is possible to express the conjugate in a single step using a gene construct of the invention encoding said conjugate, for which said construct is introduced in a vector suitable for its expression in a heterologous organism together with transcription and, optionally, translation control elements. The transcription and, optionally, translation control elements present in the expression cassette of the invention include promoters, which direct the transcription of the nucleotide sequence to which they are operatively linked and other sequences which are necessary or suitable for the transcription and its suitable regulation in time and place, for example, initiation and termination signals, cleavage sites, polyadenylation signal, replication origin, transcriptional enhancers, transcriptional silencers, etc. Said elements, as well as the vectors used for constructing the expression cassettes and the recombinant vectors according to the invention are generally chosen according to the host cells to be used.

### 4.B. Conjugates wherein components (i) and (ii) are connected by a binding pair

The conjugates of the invention wherein components (i) and (ii) are bound via the interaction of the first and second members of a binding pair connected to each of the components can be made by methods known in the art, and exemplified below in the examples.

The polypeptide comprising the projection domain of a birnavirus VP2 may contain a second member of a binding pair or may be reacted in the presence of a modifying agent which couples said second member of the binding pair to the antigenic entity. Suitable agents for coupling a first member of a binding pair to a polypeptide have been described above. In a preferred embodiment, the first member of a binding pair is a biotin-binding molecule.

The co-stimulatory molecule entity may contain a second member of a binding pair or may be reacted in the presence of a modifying agent which couples said second member of the binding pair to the antigenic entity. Suitable agents for coupling a first member of a binding pair to a polypeptide have been described above.In a preferred embodiment, the second member of a binding pair is biotin.

Incorporation of biotin into an co-stimulatory molecule may be carried out using biotinylating agents. As used herein a "biotinylating agent" refers to any molecule which is capable of adding a biotin molecule to a reactive group in a target molecule. A biotinylating agent can react with amino groups, carboxyl groups or thiol groups in the target molecule. Suitable biotinylating agents include, without limitation, Biotin-PEO-Amine (reactive with carboxyl groups), PEO-Iodoacetyl-Biotin (reactive with thiol groups), biotin-NHS, biotin-sulfoNHS, biotin-LC-NHS, biotin-LC-sulfoNHS, biotin-LC-LC-NHS, and biotin-LC-LC-sulfoNHS (reactive with amino groups). "LC" stands for "long chain," which represents a seven atom spacer between biotin and the NHS ester. Another example of a preferred biotinylating agent biotin derivative is tetrafluorophenyl polyethylene oxide biotin (TFP-PEO-biotin).

The active ester reagents are preferably used at about 0.05 - 0.5 M concentrations, and more preferably at 0.1 M concentrations, in phosphate buffered saline ("PBS") or in a solution of 9:1 PBS to dimethylsulfoxide ("DMSO"), if necessary for solubilization.

Alternatively, biotin can be added to the co-stimulatory molecule by enzymatic means using, for instance, the Biotin AviTag technology from Avidity, Inc. (Denver, Colo.). The Biotin AviTag is comprised of a unique 15 amino acid peptide that is recognized by biotin ligase, BirA that attaches biotin to a lysine residue in the peptide sequence. (Schatz, Biotechnology 1993; 11: 1138-43. The Biotin AviTag can be genetically fused to any protein of interest, allowing the protein to be tagged with a biotin molecule.

One potential drawback to the Biotin AviTag technology is the possibility of a low degree of biotinylation, because the system biotinylates the protein at a single, unique lysine residue in the tag region. To overcome any such problem, the purified tagged proteins can be modified in vitro using purified biotin ligase. Because the biotinylation is performed enzymatically, the reaction conditions are gentler, the labeling is highly specific, and the reaction is more efficient than chemical modification of the protein using biotin derivatives. Alternatively, the methods described in Jordan, et al. (Clin Diag Lab Immunol 2003;10: 339-44), can be used to produce a genetically engineered biotinylated protein.

The skilled person will appreciate that the coupling of the second member of the binding pair to the immune co-stimulatory molecule can be carried out by any method known in the art for the modification of chemical modification of molecules according to the chemical nature of said molecules. Thus, in those cases wherein the immune co-stimulatory is a polypeptide (e.g. a FcR binding polypeptide or flagelin as TLR5 agonistic ligand), any of the methods described above (chemical coupling using biotinylating agent or incorporation of a BAP peptide) are suitable for adding one or more biotin molecules to the immune co-stimulatory molecule. In those cases wherein the co-stimulatory molecule is an oligonucleotide (e.g. Poly I:C as TLR3 agonistic ligands or CpG oligonucleotides as TLR9 agonistic ligands), the immune co-stimulatory molecule can be incorporated by custom synthesis of the nucleic acids incorporating during the synthesis nucleotides modified with the second member of the binding pair. In those cases wherein the immune co-stimulatory molecule is a small organic compound (e.g, imiquimod as TLR-7 ligand), the coupling of the second member of the binding pair will depend on the chemical groups available in the co-stimulatory molecule. For instance, methods for modifying imiquimod with biotin have been described in WO09152179A1.

In those cases wherein the immune co-stimulatory molecule is lipopolysaccharide (e.g, Salmonella LPS as TLR-4 ligand), the coupling of the second member of the binding pair is typically carried out using an activated form of the second member of the binding pair capable of reacting with the glycosyl residues in the LPS molecule. For instance, WO09093250A2 describes a method for the biotinylation of LPS using biotin hydrazide.

### 5. Polynucleotides, vectors and host cells of the invention

In another aspect, the invention relates to a polynucleotide encoding a polypeotide or the invention as well as a polypeptide encoding the conjugate of the invention. A person skilled in the art will understand that the polynucleotides encoding the conjugates according to the invention will only encode the conjugates in which component (ii) has a peptide nature and which forms a single peptide chain with component (i), regardless of both the relative orientation and the fact that both components are directly connected or separated by a spacer region.

Thus, in another aspect, the invention relates to a gene construct comprising a polynucleotide of the invention. The construct preferably comprises the polynucleotide of the invention located under the operative control of sequences regulating the expression of the polynucleotide of the invention. A person skilled in the art will understand that the polynucleotides of the invention must access the nucleus of a target tissue and there be transcribed and translated to give rise to the biologically active fusion protein.

In principle, any promoter can be used for the gene constructs of the present invention provided that said promoter is compatible with the cells in which the polynucleotide is to be expressed. Thus, promoters suitable for the embodiment of the present invention include, without being necessarily limited to, constitutive promoters such as the derivatives of the genomes of eukaryotic viruses such as the polyoma virus, adenovirus, SV40, CMV, avian sarcoma virus, hepatitis B virus, the promoter of the metallothionein gene, the promoter of the herpes simplex virus thymidine kinase gene, retrovirus LTR regions, the promoter of the immunoglobulin gene, the promoter of the actin gene, the promoter of the EF-1alpha gene as well as inducible promoters in which the expression of the protein depends on the addition of a molecule or an exogenous signal, such as the tetracycline system, the NFκB/UV light system, the Cre/Lox system and the promoter of heat shock genes, the regulatable promoters of RNA polymerase II described in WO/2006/135436 as well as tissue-specific promoters. Preferably, the promoter used for expressing the polynucleotides of the invention are promoters functional in dendritic cells such as the fascin gene promoter as described by Bros et al. (J Immunol 2003;171:1825-1834), the DC-CK1, DC-STAMP and DC-SIGN gene promoters, the Dectin-2 promoter described in Morita et al., (Gene Ther 2001;8:1729-37), the CD11c gene promoter as described in Masood, R., et al. (Int J Mol Med 2001;8:335-343) and Somia, N. V., et al. (Proc Acad Sci USA 1995;92:7570-7574).

Other examples of promoters which are tissue-specific include the promoter of the albumin gene (Miyatake et al., J Virol 1997;71:5124-32), the core promoter of hepatitis virus (Sandig et al, Gene Ther 1996;3:1002-9), the promoter of the alpha-fetoprotein gene (Arbuthnot et al., Hum.GeneTher 1996;7:1503-14), and the promoter of the globulin-binding protein which binds to thyroxine (Wang, L., et al., Proc Natl Acad Sci USA 1997;94:11563-11566).

The polynucleotides of the invention or the gene constructs forming them can form part of a vector. Thus, in another aspect, the invention relates to a vector comprising a polynucleotide or a gene construct of the invention. A person skilled in the art will understand that there is no limitation as regards the type of vector which can be used because said vector can be a cloning vector suitable for propagation and for obtaining the polynucleotides or suitable gene constructs or expression vectors in different heterologous organisms suitable for purifying the conjugates. Thus, suitable vectors according to the present invention include expression vectors in prokaryotes such as pUC18, pUC19, Bluescript and their derivatives, mp18, mp19, pBR322, pMB9, CoIE1, pCR1, RP4, phages and shuttle vectors such as pSA3 and pAT28, expression vectors in yeasts such as vectors of the type of 2 micron plasmids, integration plasmids, YEP vectors, centromeric plasmids and the like, expression vectors in insect cells such as the pAC series and pVL series vectors, expression vectors in plants such as vectors of expression in plants such as pIBI, pEarleyGate, pAVA, pCAMBIA, pGSA, pGWB, pMDC, pMY, pORE series vectors and the like and expression vectors in superior eukaryotic cells based on viral vectors (adenoviruses, viruses associated to adenoviruses as well as retroviruses and lentiviruses) as well as non-viral vectors such as pSilencer 4.1-CMV (Ambion), pcDNA3, pcDNA3.1/hyg pHCMV/Zeo, pCR3.1, pEF1/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1.

The vector of the invention can be used to transform, transfect or infect cells which can be transformed, transfected or infected by said vector. Said cells can be prokaryotic or eukaryotic. By way of example, the vector wherein said DNA sequence is introduced can be a plasmid or a vector which, when it is introduced in a host cell, is integrated in the genome of said cell and replicates together with the chromosome (or chromosomes) in which it has been integrated. Said vector can be obtained by conventional methods known by the persons skilled in the art (Sambrook et al., Molecular Cloning: A Laboratory Manual, 2001), mentioned above).

Therefore, in another aspect, the invention relates to a cell comprising a conjugate, a polynucleotide, a gene construct or a vector of the invention, for which said cell has been able to be transformed, transfected or infected with a construct or vector provided by this invention. The transformed, transfected or infected cells can be obtained by conventional methods known by persons skilled in the art (Sambrook *et al.,* 2001, mentioned above). In a particular embodiment, said host cell is an animal cell transfected or infected with a suitable vector.

Host cells suitable for the expression of the conjugates of the invention include, without being limited to, mammal, plant, insect, fungal and bacterial cells. Bacterial cells include, without being limited to, Gram-positive bacterial cells such as species of the Bacillus, Streptomyces, *Listeria* and Staphylococcus genus and Gram-negative bacterial cells such as cells of the Escherichia, *Salmonella* and Pseudomonas genus. Fungal cells preferably include cells of yeasts such as *Saccharomyces, Pichia pastoris* and *Hansenula polymorpha.* Insect cells include, without being limited to, Drosophila and Sf9 cells. Plant cells include, among others, cells of crop plants such as cereals, medicinal, ornamental or bulbous plants. Suitable mammal cells in the present invention include epithelial cell lines (human, ovine, porcine, etc.), osteosarcoma cell lines (human, etc.), neuroblastoma cell lines (human, etc.), epithelial carcinomas (human, etc.), glial cells (murine, etc.), hepatic cell lines (from monkey, etc.), CHO (Chinese Hamster Ovary) cells, COS cells, BHK cells, HeLa cells, 911, AT1080, A549, 293 or PER.C6, NTERA-2 human ECC cells, D3 cells of the mESC line, human embryonic stem cells such as HS293, BGV01, SHEF1, SHEF2, HS181, NIH3T3 cells, 293T, REH and MCF-7 and hMSC cells.

### 6. Therapeutic uses of the conjugates according to the invention

The authors of the present invention have observed that the polypeptides of the invention is capable of inducing the activation of an immune response *in vivo* against the birnavirus VP2 polypeptide. These results show that a polypeptide comprising a birnavirus VP2 P domain and lacking the AA' hairpin may be capable of inducing the activation of an immune response *in vivo* against the birnavirus VP2 polypeptide. Moreover, the authors of the present invention have observed that the conjugates of the invention are capable of inducing the activation of an immune response *in vivo* against the birnavirus VP2 polypeptide. These results show that a polypeptide comprising a birnavirus VP2 P domain lacking the AA' hairpin and a first member of a binding pair can be used to prepare immunoreactive complexes with co-stimmulatory molecules coupled to a second member of said binding pair which could be included in prophylactic or therapeutic vaccine formulations against infections caused by birnaviruses.

Thus, in another aspect, the invention relates to a pharmaceutical composition, or pharmaceutical composition of the invention comprising an immunologically effective amount of a polypeptide or conjugate of the invention and at least one pharmacologically acceptable carrier or adjuvant. In yet another aspect, the invention relates to a conjugate or pharmaceutical composition according to the invention for use in medicine.

The term "immunogenically effective amount" has its usual meaning in the art, *i.e.* an amount of an immunogen which is capable of inducing a significant immune response against pathogenic agents that share immunological features with the immunogen.

"Adjuvant" is understood as any substance intensifying the effectiveness of the pharmaceutical composition of the invention. Suitable adjuvants include, without limitation, adjuvants formed by aluminum salts (alum), such as aluminum hydroxide, aluminum phosphate, aluminum sulfate, etc, formulations of oil-in-water or water-in-oil emulsions such as complete Freund's Adjuvant (CFA) as well as the incomplete Freund's Adjuvant (IFA); mineral gels; block copolymers, Avridine™, SEAM62, adjuvants formed by components of the bacterial cell wall such as adjuvants including liposaccharides (e.g., lipid A or Monophosphoryl Lipid A (MLA), trehalose dimycolate (TDM), and components of the cell wall skeleton (CWS), heat shock proteins or the derivatives thereof, adjuvants derived from ADP-ribosylating bacterial toxins, which include diphtheria toxin (DT), pertussis toxin (PT), cholera toxin (CT), *E.coli* heat-labile toxins (LT1 and LT2), *Pseudomonas* Endotoxin A and exotoxin, *B. cereus* exoenzyme B, *B. sphaericus* toxin, *C*. *botulinum* toxins C2 and C3, C. *limosum* exoenzyme as well as the toxins of *C*. *perfringens, C. spiriforma* and *C*. *difficile, S. aureus,* EDIM and mutants of mutant toxins such as CRM-197, non-toxic mutants of diphtheria toxin; saponins such as ISCOMs (immunostimulating complexes), chemokines, quimiokines and cytokines such as interleukins (IL-I IL-2, IL-4, IL-5, IL-6, IL-7, IL-8, IL-12, etc), interferons (such as the interferon gamma) macrophage colony stimulating factor (M-CSF), tumor necrosis factor (TNF), defensins 1 or 2, RANTES, MIP1-alpha, and MEP-2, muramyl peptides such as N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-s-n-glycero-3-hydroxyphosphoryloxy)-ethylamine (MTP-PE) etc; adjuvants derived from the family of CpG molecules, CpG dinucleotides and synthetic oligonucleotides which comprise CpG motifs, *C*. *limosum* exoenzyme and synthetic adjuvants such as PCPP, the cholera toxin, *Salmonella* toxin, alum and the like, aluminum hydroxide, N-acetyl-muramyl-L-threonyl-D-isoglutamine (thr-MDP), N-acetyl-nor-muramyl-L-alanyl-D-isoglutamine, MTP-PE and RIBI, containing three components extracted from bacteria, monophosphoryl lipid A, trehalose dimycolate and cell wall skeleton (MPL+TDM+CWS) in a squalene emulsion at 2% Tween 80. Other examples of adjuvants include DDA (dimethyl dioctadecyl ammonium bromide) and QuilA.

The term "carrier" refers to a diluent or excipient with which the active ingredient is administered. Such pharmaceutical carriers can be sterile liquids, such as water and oils, including those of petroleum, animal, plant or synthetic origin, such as peanut oil, soybean oil, mineral oil, sesame oil and the like. Water or aqueous solutions of saline solution and aqueous dextrose and glycerol solutions, particularly for injectable solutions, are preferably used as carriers. Suitable pharmaceutical carriers are described in "Remington's Pharmaceutical Sciences" by E.W. Martin, 1995. Preferably, the carriers of the invention are approved by a regulatory agency of the Federal or a state government or listed in the United States Pharmacopoeia or another generally recognized pharmacopeia for use in animals, and more particularly in humans.

For use in medicine, the pharmaceutical compositions of the invention can be administered by any route, including, without limitation, oral, intravenous, intramuscular, intrarterial, intramedullary, intrathecal, intraventricular, transdermal, subcutaneous, intraperitoneal, intranasal, enteral, topical, sublingual or rectal route. A review of the different forms for the administration of active ingredients, of the excipients to be used and of the processes for manufacturing them can be found in Tratado de Farmacia Galénica, C. Faulí i Trillo, Luzán 5, S.A. de Ediciones, 1993 and in Remington's Pharmaceutical Sciences (A.R. Gennaro, Ed.), 20th edition, Williams & Wilkins PA, USA (2000). Examples of pharmaceutically acceptable carriers are known in the state of the art and include phosphate buffered saline (PBS) solutions, water, emulsions, such as oil/water emulsions, different types of wetting agents, sterile solutions, etc. The compositions comprising said carriers can be formulated by conventional methods known in the state of the art.

In another aspect, the invention relates to a polypeptide, conjugate or pharmaceutical composition according to the invention for use in a method of treatment of a disease which requires the generation of an immune response against a birnavirus VP2. Alternatively, the invention relates to the use of a conjugate or pharmaceutical composition according to the invention for the manufacture of a medicament for the treatment of a disease which requires the generation of an immune response against a birnavirus VP2. Alternatively, the invention relates to a method for the treatment of a disease which requires the generation of an immune response against a birnavirus VP2 in a subject in need thereof which comprises the administration to said subject a conjugate according to the invention.

The skilled person will appreciate that the disease to be treated will require an adequate conjugate which comprises the polypeptide comprising the VP2 P domain derived from the birnavirus which is causing the disease. Thus, in further aspects, the invention relates to therapeutic uses of the conjugate and pharmaceutical composition according to the invention wherein
(i) wherein the polypeptide comprising the projection domain of a birnavirus VP2 is dertived from IBDV VP2, then then the disease which requires the generation of an immune response against said polypeotide is an infectious disease caused by IBDV the pathogenic microorganism, such as infectious bursal disease (IBD) (also known as Gumboro disease) which is characterized by bursal inflammation, hemorrhage, lymphocytolysis, atrophy, depression, and liver necrosis or
(ii) wherein the polypeptide comprising the projection domain of a birnavirus VP2 is dertived from IPNV VP2, then then the disease which requires the generation of an immune response against said polypeptide is an infectious disease caused by IPNV, such as infectious pancreatic necrosis disease (IPN).

In another aspect, the invention relates to the use of a composition or kit-of-parts of the invention or a pharmaceutical composition of the invention for the manufacture of a vaccine.

As used herein, the term "vaccine" refers to a formulation which contains a composition according to the present invention and which is in a form that is capable of being administered to a vertebrate and which induces a protective immune response sufficient to prevent and/or ameliorate an infection and/or to reduce at least one symptom of an infection and/or to enhance the efficacy of another dose of composition of the invention. As it will be understood, the immune response generated by the vaccine may be a humoral or a cellular immune response.

The expression "cellular immune response", is used herein to describe an immune response against foreign antigen(s) that is mediated by T-cells and their secretion products.

The "humoral immune response", is used herein to describe an immune response against foreign antigen(s) that is mediated by antibodies produced by B-cells.

The vaccine is systemically or locally administered. The vaccine can be administered by means of a single administration, or with a boost by means of multiple administrations as has been previously described for the administration of the compositions of the invention.

The immunogenic composition and vaccines according to the invention can be presented as a single formulation (for example, as a tablet or capsule comprising a fixed amount of each component) or, otherwise, can be presented as separate formulations for subsequent combination for joint, sequential or separate administration. The compositions of the invention also contemplate the formulation as a kit of parts wherein the components are formulated separately but are packaged in the same container.

The expert in the art will appreciate that the formulation of the first and second component of the compositions of the invention can be similar, in other words, formulated in a similar way (for example, in tablets or in pills), allowing administration by the same route. In an embodiment wherein the different components of the invention are formulated separately, the two components can be presented in a blister pack. Each blister will contain the medicaments to be consumed throughout the day. If the medicaments need to be administered several times a day, the medicaments corresponding to each administration can be arranged in separate sections of the blister pack, preferably noting on each section of the blister pack the time of day when they need to be administered. Alternatively, the components of the composition of the invention can be formulated in a different manner so that the different components are administered differently. Thus, it is possible, for example, for the first component to be formulated as a tablet or capsule for oral administration and for the second component to be formulated for intravenous administration.

The injection of protein antigens typically is used to elicit immune responses in a recipient animal. However, the invention also provides methods of delivering antigen to APCs by DNA injection. A commonly used technique is to inject DNA expression vectors, encoding an antigenic protein, into muscle. Reports suggest that the protein antigen is expressed by muscle cells but that the antigen is not presented to the immune system by these cells. Instead, it is believed that specialized APCs, for example, macrophages and dendritic cells, migrate to the site of injection, pick up and present the antigen through a process that has not yet been elaborated. Use of Fc-antigen fusion protein expression vectors make this process more efficient because the secreted fusion protein binds more efficiently to APCs than native antigen protein.

One consequence of the DNA injection approach is that it can often result in the generation of both humoral and cellular responses. Typically, proteins administered exogenously have a more difficult time entering the pathway for presentation on MHC class I molecules. Nevertheless, administration of the Fc fusion proteins of the invention enhance the generation of cytotoxic cells, likely through MHC class I presentation of the preselected exogenous antigen. Combinations of DNA immunization and protein immunization also can work synergistically to first prime the immune system and then boost the level of response in the form of both antibody production and cytotoxic cellular responses. Co-administration of Fc-adjuvant fusion protein, for example, Fc-IL-2, Fc-GMCSF, Fc-IL-12, and Fc-Flt3 ligand, together with the Fc-antigen fusion protein ensures co-localization of the fusion proteins to the same cellular compartment of the APCs, thereby stimulating a more potent immune response against the preselected antigen.

The compositions of the present invention (i.e., conjugates or nucleic acid sequences encoding such conjugates) may be provided to an animal by any suitable means, directly (e.g., locally, as by injection, implantation or topical administration to a tissue locus) or systemically (e.g., parenterally or orally). Where the composition is to be provided parenterally, such as by intravenous, subcutaneous, ophthalmic, intraperitoneal, intramuscular, buccal, rectal, vaginal, intraorbital, transdermal, intracerebral, intracranial, intraspinal, intraventricular, intrathecal, intracisternal, intracapsular, intranasal or by aerosol administration, the composition preferably comprises part of an aqueous or physiologically compatible fluid suspension or solution. Thus, the carrier or vehicle is physiologically acceptable so that in addition to delivery of the desired composition to the patient, it does not otherwise adversely affect the patient's electrolyte and/or volume balance. The fluid medium for the agent thus can comprise normal physiologic saline (e.g., 9.85% aqueous NaCl, 0.1 SM, pH 7-7.4). Preferred dosages of the conjugates per administration are within the range of 50 ng/m 2 to 1 g/m 2, more preferably 5 mu g/m 2 to 200 mg/m 2, and most preferably 0.1 mg/m 2 to 50 mg/m 2. Preferred dosages of nucleic acids encoding the conjugates per administration are within the range of 1 mu g/m 2 to 100 mg/m 2, more preferably 20 mu g/m 2 to 10 mg/m 2, and most preferably 400 mu g/m 2 to 4 mg/m 2.

It is contemplated that maximal immunization may be achieved by performing numerous separate immunizations, for example, one to three inoculations about 3 weeks to six months apart. Furthermore, as discussed above, maximal immune responses can be achieved under certain circumstances by alternating between the administration of conjugates and nucleic acids encoding such conjugates. The optimal modes of administration, dosages and booster regimes may be determined by routine experimentation well within the level of skill in the art.

### 7. Further compositions of the invention

In another embodiment, the invention provides a conjugate comprising a birnavirus VP2 P domain lacking the AA' hairpin region and a first member of a binding pair wherein said first member of a binding pair is not a region from said birnavirus VP2.

The terms "conjugate", "birnavirus", "VP2", "P domain", "AA' hairpin region" and "first member of a binding pair" have been described in detail above.

In a preferred embodiment, the first member of the binding pair is a biotin-binding region and, more preferably, avidin or a functionally equivalent variant thereof. Suitable functionally equivalent variants of avidin have been described in detail above.

In a preferred embodiment, the conjugate further comprises one or more peptide tags. Suitable tags for use in the conjugates have described in detail above. Preferably, the tag is selected from the group consisting of an hexahistidine tag, a FLAG tag or a combination thereof.

As described above, the conjugate may be formed by the chemical cross-linking of the polypeptide comprising a birnavirus VP2 P domain lacking the AA' hairpin region and the first member of a binding pair. Alternatively, the conjugate may result from the genetic cross-linking of both regions, in which case the birnavirus VP2 P domain and the avidin or the functionally equivalent variant thereof form a fusion protein.

In further embodiments, the invention relates to a polynucleotide encoding a fusion protein as above, to a vector comprising a polynucleotide as defined above or a host cell comprising a conjugate, a polynucleotide or a vector as defined above.

### 8. Immunogenic cell-based compositions of the invention

Another therapy approach is to take advantage of the normal antigen presenting cells role as an immune educator. As has been previously mentioned, antigen presenting cells capture virus antigens among others and present them to T cells to recruit their help in an initial T cell immune response. Due to the fact that an antigen alone may not be enough to generate an immune response, it is possible to contact an immature antigen presenting cells with a conjugate according to the invention which results in the activation and maturation of the antigen presenting cells, the capture of the antigen or antigens found in the antigenic entity and the presentation thereof in the surface associated to the major histocompatibility antigen. These cells thus activated and maturated can be administered to the patient, such that the presentation of the antigens to the immune system of the patient occurs, which eventually results in the generation of an immune response mediated by T cells.

Thus, in another aspect, the invention relates to *an in vitro* method for obtaining an immunogenic antigen presenting cell specific for a given antigenic entity comprising the steps of:
(i) contacting an immature antigen presenting cell with a conjugate according to the invention under conditions adequate for stimulation of the antigen presenting and
(ii) recovering the immunogenic antigen presenting cell.

The term "antigen presenting cell" (APC), as used herein, refers to a class of cells capable of presenting one or more antigens in the form of peptide-MHC complex recognizable by specific effector cells of the immune system, and thereby inducing an effective cellular immune response against the antigen or antigens being presented. APC suitable for use in the present invention include both professional APC such as dendritic cells (DC), macrophages and B-cells as well as non-professional APC such as fibroblasts, thymic epithelial cells, thyroid epithelial cells, glial cells, pancreatic beta cells and vascular endothelial cells. In a preferred embodiment, the APC are dendritic cells. Preferably, the APC for use in the methods of the inventions are dendritic cells, since these are the only APC having the capacity to present antigens in an efficient amount to activate naive T-cells for cytotoxic T-lymphocyte (CTL) responses.

The term "immunogenic", when used herein to refer to the APCs, refers to phenotypically mature antigen presenting cell with and effector function of immunogenicity (Reis e Sousa C., Nature reviews, 2006; 6:476-483). A phenotiypically mature antigen presenting cell is an APC expressing high cell-surface levels of MHC molecules, CD40, CD80, CD83 and CD86. An effector function of immunogenicity refers to the ability to prime an immune response. These cells have to be distinguished from tolerogenic APCs, which are cells that, in the absence of deliberate exposure to maturation signals — can tolerize peripheral CD4+ and CD8+ T cells by inducing deletion, anergy or regulation, depending on the model system studied.

The term "dendritic cells (DCs)" refers to a diverse population of morphologically similar cell types found in a variety of lymphoid and non-lymphoid tissues (Steinman Ann. Rev Immunol 1991;9:271-296). Dendritic cells constitute the most potent and preferred APCs in the organism. While the dendritic cells can be differentiated from monocytes, they possess distinct phenotypes. For example, a particular differentiating marker, CD14 antigen, is not found in dendritic cells but is possessed by monocytes. Also, mature dendritic cells are not phagocytic, whereas the monocytes are strongly phagocytotic cells. It has been shown that mature DCs can provide all the signals necessary for T cell activation and proliferation.

Dendritic cells can be isolated or generated from blood or bone marrow, or secondary lymphoid organs of the subject, such as but not limited to spleen, lymph nodes, tonsils, Peyer's patches of the intestine, and bone marrow, by any of the methods known in the art. Preferably, DCs used in the methods of the invention are (or terminally differentiated) dendritic cells. The source of dendritic cells is preferably human blood monocytes.

Immune cells obtained from such sources typically comprise predominantly recirculating lymphocytes and macrophages at various stages of differentiation and maturation. Dendritic cell preparations can be enriched by standard techniques (see e.g., Current Protocols in Immunology, 7.32.1-7.32.16, John Wiley and Sons, Inc., 1997) such as by depletion of T cells and adherent cells, followed by density gradient centrifugation. DCs may optionally be further purified by sorting of fluorescence-labeled cells, or by using anti-CD83 MAb magnetic beads. Alternatively, a high yield of a relatively homogenous population of DCs can be obtained by treating DC progenitors present in blood samples or bone marrow with cytokines, such as granulocyte-macrophage colony stimulating factor (GM-CSF) and interleukin 4 (IL-4). Under such conditions, monocytes differentiate into dendritic cells without cell proliferation. Further treatment with agents such as TNF alpha stimulates terminal differentiation of DCs.

Alternatively, the antigen-presenting cells (APCs), including but not limited to macrophages, dendritic cells and B-cells, can be obtained by production *in vitro* from stem and progenitor cells from human peripheral blood or bone marrow as described by Inaba et al. (J Exp Med;1992:176 1693-1702).

APCs and, in particular, dendritic cells, obtained in this way characteristically express the cell surface marker CD83. In addition, such cells characteristically express high levels of MHC class II molecules, as well as cell surface markers CD1 alpha, CD40, CD86, CD54, and CD80, but lose expression of CD14. Other cell surface markers characteristically include the T cell markers CD2 and CD5, the B cell marker CD7 and the myeloid cell markers CD13, CD32 (Fc gamma R II), CD33, CD36, and CD63, as well as a large number of leukocyte-associated antigens.

Optionally, standard techniques, such as morphological observation and immunochemical staining, can be used to verify the presence of APCs and, in particular, dendritic cells. For example, the purity of APCs and, in particular, dendritic cells, can be assessed by flow cytometry using fluorochrome-labeled antibodies directed against one or more of the characteristic cell surface markers noted above, e.g., CD83, HLA-ABC, HLA-DR, CD1 alpha, CD40, and/or CD54. This technique can also be used to distinguish between immature and mature DCs, using fluorochrome-labeled antibodies directed against CD 14, which is present in immature, but not mature, DCs.

APCs and, in particular, dendritic cell precursors may be obtained from a healthy subject or a subject known to be suffering from a disease associated with the expression of a particular antigen. Such DC precursors may be allogeneic or autologous.

Once APCs precursors are obtained, they are cultured under appropriate conditions and for a time sufficient to expand the cell population and maintain the APCs in a state for optimal antigen uptake, processing and presentation. In one preferred approach to culture of APC precursors, APCs are generated from such APCs precursors by culture *ex vivo* in serum free or protein-free medium for 40 hours, in the absence of exogenously added cytokines, as detailed in WO0127245.

Preferred aspects of APC isolation and culture include the use of culture medium lacking exogenously supplied cytokines and culture under serum-free conditions in a manner effective to result in the generation of antigen-loaded superactivated DC, which are cells that have already processed an antigen and have the ability to present the antigen to the immune cells and quickly generate antigen-specific immune responses, e. g., CTL-mediated T cell responses to tumor antigens.

Dendritic cells can be preserved by cryopreservation either before or after exposure to the compositions or kit-of-parts of the invention.

In step (i) of the method of obtaining immunogenic APC of the invention, the immature APC are contacted with a conjugate according to the invention. Typically, the contacting step comprises the contacting/incubating of the immature APC with the conjugate for sufficient time. In one embodiment, sensitization may be increased by contacting the APCs with heat shock protein(s) (hsp) noncovalently bound to the composition. It has been demonstrated that hsps noncovalently bound to antigenic molecules can increase APC sensitization.

The activation of the APC can be detected by contacting the APC with T cell clones expressing a T-cell receptor specific for the antigenic peptide present in the composition and measuring the proliferation of the T-cells, usually by measuring the incorporation of a labeled nucleotide analog.

Once the APC have been sensitized with the antigen, the cells are isolated in order to obtain the antigen-primed APC. Cell surface markers can be used to isolate the cells necessary to practice the methods of this invention. For example, DCs express MHC molecules and costimulatory molecules (e.g., B7-1 and B7-2). The expression of surface markers facilitates identification and purification of these cells. These methods of identification and isolation include FACS, column chromatography and the like.

Labeling agents which can be used to label cell antigen include, but are not limited to monoclonal antibodies, polyclonal antibodies, proteins, or other polymers such as affinity matrices, carbohydrates or lipids. Detection proceeds by any known method, such as immunoblotting, western blot analysis, tracking of radioactive or bioluminescent markers, capillary electrophoresis, or other methods which track a molecule based upon size, charge or affinity.

Thus, in another aspect, the invention relates to an APC obtainable by the method previously mentioned.

The antigen-loaded immunogenic APC obtained using the method of the present invention can be used to activate CD8+ T cells and/or CD4+ T*-*cells *in vitro* or can be introduced directly in a subject to activate the T cells *in vivo.* Thus, in further aspects, the invention relates to an APC obtainable by the method of the invention for use in medicine as well as to a vaccine or immunogenic composition comprising the APC obtainable by the method of the invention. It will be understood that, for the purposes of medical uses, the cells may originate from the same individual which is to be treated (autologous transplantation) or from a different individual (allogeneic transplantation). In allogeneic transplantation, the donor and recipient are matched based on similarity of HLA antigens in order to minimize the immune response of both donor and recipient cells against each other.

The APCs according to the invention or the pharmaceutical compositions comprising said APCs or DCs can be used in a method for the treatment of treatment of a disease which requires the generation of an immune response against the birnavirus VP2.

CD8⁺ T cells educated *in vitro* can be introduced into a subject where they are cytotoxic against target cells bearing antigenic peptides corresponding to T cells are activated to recognize them on class I MHC molecules. These target cells are typically pathogen-infected cells which express unique antigenic peptides on their MHC class I surfaces.

Similarly, CD4⁺ helper T cells, which recognize antigenic peptides in the context of MHC class II, can also be stimulated by the APCs of the invention, which present antigenic peptides both in the context of class I and class II MHC. Helper T cells also stimulate an immune response against a target cell. As with cytotoxic T cells, helper T cells are stimulated with the antigen-loaded APCs *in vitro* or *in vivo.*

Thus, in another aspect, the invention relates to an antigen-presenting cell according to the invention, for use in a method for induction of a cytotoxic cell response against the antigen. In another aspect, the invention relates to an antigen-presenting cell according to the invention for use in a method for induction of a cytotoxic cell response against an disease caused by a birnavirus.

The immunogenicity of the antigen-presenting cells or educated T cells produced by the methods of the invention can be determined by well known methodologies including, but not limited to the following:
- ⁵¹Cr-release lysis assay (or equivalent) for CTL function,
- Assay for cytokines released by T cells upon contacting modified APCs
- In *vitro* T-cell education
- Transgenic animal models
- Proliferation Assays which measures the capacity of T cells to proliferate in response to reactive compositions.
- Monitoring TCR Siqnal Transduction Events.

The APC cells can be used for the treatment of different diseases depending on the type of birnavirus VP2 which form part of the compositions used for the sensitization of the antigen-presenting cells. Birnavirus VP2 suitable for sensitization have been described previously. Thus, in another aspect, the invention relates to the antigen-presenting cells of the invention for use in the treatment of a disease which requires the generation of an immune response against the birnavirus VP2. Alternatively, in another aspect, the invention relates to the use of an antigen-presenting cells of the invention in the manufacture of a medicament for the treatment of a disease which requires the generation of an immune response against the birnavirus VP2. Alternatively, the invention relates to a method for the treatment of a disease that requires the generation of an immune response against the birnavirus VP2 in a subject which comprises the administration to said subject of an antigen-presenting cells of the invention.

If the antigen used for preparing the antigen-presenting cells is the projection domain of a birnavirus VP2 and lacking the AA' hairpin region derived from IBDV, then the disease caused by a birnavirus infection that can be treated or prevented using the cells of the invention is infectious bursal disease.

If the antigen used for preparing the antigen-presenting cells is the projection domain of a birnavirus VP2 and lacking the AA' hairpin region derived from IPNV, then the disease caused by a birnavirus infection that can be treated or prevented using the cells of the invention is infectious pancreatic necrosis.

Preferably, the methods of treatment of the present invention comprised the so-called adoptive immunotherapy. The term "adoptive immunotherapy" refers to a therapeutic approach for treating a disease caused by birnavirus infection in which immune cells are administered to a host with the aim that the cells mediate either directly or indirectly specific immunity to (i.e., mount an immune response directed against) the undesired cells. The immune cells can be derived from a different organism/host (exogenous immune cells) or can be cells obtained from the subject organism (autologous immune cells).

The immune cells are typically activated *in vitro* by a particular antigen (in this case the antigenic entity used in the compositions of the invention) applying any of the techniques mentioned above for the activation of APC *in vitro.* Methods of performing adoptive immunotherapy are well known to those of skill in the art (see, e g, US Pat Nos 5,081,029, 5,985,270, 5,830,464, 5,776,451, 5,229,115, 690,915, and the like). The invention contemplates numerous modalities of adoptive immunotherapy. In one embodiment, the DC (e.g. isolated from the patient or autologous dendritic cells) are pulsed with the compositions of the invention and then injected back into the subject where they present and activate immune cells *in vivo.* In yet another embodiment, the DC are pulsed with the compositions of the invention and then used to stimulate peripheral blood lymphocytes or TIL in culture and activate CTLs targeted against the antigenic entity that are then infused into the patient. Similarly, fibroblasts, and other APCs, or tumor cells are pulsed with the compositions of the invention and used to activate tumor cells or PBLs *ex vivo* to produce CTLs directed against the antigenic entity that can then be infused into a patient.

Inoculation of the activated cells is preferably through systemic administration. The cells can be administered intravenously through a central venous catheter or into a large peripheral vein. Other methods of administration (for example, direct infusion into an artery) are within the scope of the invention.

The APCs of the invention and, in particular, the dendritic cells of the invention, can be provided in a formulation which is suitable for administration to a patient, e.g., intravenously. APCs and, in particular, DCs of the invention, that are suitable for administration to a patient are referred to herein as a "vaccine", "APC vaccine" or "DC vaccine." A vaccine or DC vaccine may further comprise additional components to help modulate the immune response, or it may be further processed in order to be suitable for administration to a patient. Methods of intravenous administration of dendritic cells are known in the art, and one of skill in the art will be able to vary the parameters of intravenous administration in order to maximize the therapeutic effect of the administered DCs.

Thus, APCs or DCs are administered to a subject in any suitable manner, often with at least one pharmaceutically acceptable carrier. The suitability of a pharmaceutically acceptable carrier is determined in part by the particular composition being administered, as well as by the particular method used to administer the composition. Most typically, quality control tests (e.g., microbiological assays clonogenic assays, viability tests), are performed and the cells are reinfused back to the subject, in some cases preceded by the administration of diphenhydramine and hydrocortisone. See, e.g., Korbling et al. Blood 1986;67:529-532 and Haas et al. Exp. Hematol 1990;18:94-98*.* Formulations suitable for parenteral administration, such as, for example, intravenous administration, include aqueous isotonic sterile injection solutions which can contain antioxidants, buffers, bacteriostats, and solutes that render the formulation isotonic with the blood of the intended recipient, as well as aqueous and non-aqueous sterile suspensions that can include suspending agents, solubilizers, thickening agents, stabilizers, and preservatives. Generally, APCs of the invention can be administered to a subject at a rate determined by the effective dose, the toxicity of the cell type (e.g., the LD-50), and the side-effects of the cell type at various concentrations, as appropriate to the mass and overall health of the subject as determined by one of skill in the art. Administration can be accomplished via single or divided doses. The APCs of the invention can supplement other treatments for a disease or disorder, including, for example, conventional radiation therapy, cytotoxic agents, nucleotide analogues and biologic response modifiers.

The dose of the APCs administered to a patient, in the context of the present invention should be sufficient to trigger a beneficial therapeutic response in the patient over time, or to inhibit growth of cancer cells, or to inhibit infection. Thus, cells are administered to a patient in an amount sufficient to elicit an effective CTL response to the virus or tumor antigen and/or to alleviate, reduce, cure or at least partially arrest symptoms and/or complications from the disease or infection. An amount adequate to accomplish this is defined as a "therapeutically effective dose." The dose will be determined by the activity of the APC produced and the condition of the patient, as well as the body weight or surface area of the patient to be treated. The size of the dose also will be determined by the existence, nature, and extent of any adverse side-effects that accompany the administration of a particular cell in a particular patient. In determining the effective number of cells to be administered in the treatment or prophylaxis of diseases such as cancer (e.g., metastatic melanoma, prostate cancer, etc.), the physician needs to evaluate circulating plasma levels, CTL toxicity, progression of the disease, and the induction of immune response against any introduced cell type.

Prior to infusion, blood samples are obtained and saved for analysis. Generally at least about 10⁴ to 10⁶ and typically, between 10⁸ and 10¹⁰ cells are infused intravenously or intraperitoneally into a 70 kg patient over roughly 60-120 minutes. Preferably, cell numbers of at least 10⁷ for each vaccination point are used. The injections may be e.g. 4 times repeated in a 2 weeks interval and should be given preferably near lymph nodes by intradermal or subcutaneous injections. Booster injections may be performed after a 4-week pause. Vital signs and oxygen saturation by pulse oximetry are closely monitored. Blood samples are obtained 5 minutes and 1 hour following infusion and saved for analysis. Cell reinfusion is repeated roughly every month for a total of 10-12 treatments in a one year period. After the first treatment, infusions can be performed on a outpatient basis at the discretion of the clinician. If the reinfusion is given as an outpatient, the participant is monitored for at least 4 hours following the therapy. For administration, cells of the present invention can be administered at a rate determined by the LD-50 (or other measure of toxicity) of the cell type, and the side-effects of the cell type at various concentrations, as applied to the mass and overall health of the patient. Administration can be accomplished via single or divided doses. In some regimens, patients may optionally receive in addition a suitable dosage of a biological response modifier including but not limited to the cytokines IFN-α, IFN-γ, IL-2, IL-4, IL-6, TNF or other cytokine growth factor, antisense TGF-β, antisense IL-10, and the like.

### 9. Further aspects of the invention

[1] A polypeptide comprising a birnavirus VP2 P domain wherein said birnavirus VP2 P domain lacks the AA' hairpin region.

[2] A polypeptide as defined in [1] further comprising one or more tags.

[3] A polypeptide as defined in [2] wherein the tag is an hexahistidine tag or a FLAG tag.

[4] A polypeptide as defined in any of [1] to [3] wherein the birnavirus is IBDV.

[5] A conjugate comprising
(i) a first component comprising the projection domain of a birnavirus VP2 wherein said birnavirus VP2 projection domain lacks the AA' hairpin region and
(ii) a second component which is an immune co-stimulatory agent wherein said component (ii) is not a region of said birnavirus VP2.

[6] A conjugate as defined in [5] wherein said components (i) and (ii) are connected via the interaction between a first member of a binding pair coupled to component (i) and a second member of a binding pair coupled to component (ii).

[7] A conjugate as defined in [6] wherein the first member of the binding pair is avidin or a functionally equivalent variant thereof and the second member of the binding pair is biotin or wherein the first member of the binding pair is biotin and the second member of the binding pair is avidin or a functionally equivalent variant thereof.

[8] A conjugate as defined in [5] wherein components (i) and (ii) are covalently coupled.

[9] A conjugate as defined in [8] wherein components (i) and (ii) form a fusion protein.

[10] A conjugate as defined in any of [5] to [9] wherein the immune co-stimulatory agent is a molecule capable of binding to an antigen-presenting cell.

[11] A conjugate as defined in [10] wherein the molecule capable of binding to an antigen-presenting cell is selected from the group consisting of a TLR agonist and a Ig Fc region.

[12] A conjugate as defined in [11] wherein the TLR agonist is selected from the group of consisting of flagellin and a CpG oligonucleotide.

[13] A conjugate as defined in [11] wherein the Ig Fc region is the IgY Fc region.

[14] A conjugate as defined in any of [5] to [13] further comprising one or more tags.

[15] A conjugate as defined in [14] wherein the tag is an hexahistidine tag of a FLAG tag.

[16] A conjugate as defined in any of [5] to [15] wherein the birnavirus is IBDV or IPNV.

[17] A polynucleotide encoding a polypeptide as defined in any of [1] to [4] or [9] to [16].

[18] A vector comprising a polynucleotide as defined in [17].

[19] A host cell comprising a polypeptide as defined in any of [1] to [4], a conjugate according to any of [5] to [16], a polynucleotide as defined in [17] or a vector as defined in [18].

[20] A vaccine composition comprising an immunologically active amount of a polypeptide as defined in any of [1] to [4], a conjugate according to any of [5] to

[16], a polynucleotide as defined in [17] or a vector as defined in [18].

[21] A polypeptide as defined in any of [1] to [4], a conjugate as defined in any of [5] to [16], a polynucleotide as defined in [17] or a vector as defined in [18] for use in medicine.

[22] A polypeptide as defined in any of [1] to [4], a conjugate as defined in any of [5] to [16], a polynucleotide as defined in [17] or a vector as defined in [18] for use in a method of treating a disease caused by a birnavirus infection.

[23] A polypeptide, conjugate, polynucleotide or vector for use according to [22] wherein
(i) the disease caused by a birnavirus infection is infectious bursal disease in which case the component comprising the projection domain of a birnavirus VP2 and lacking the AA' hairpin region derives from IBDV or
(ii) the disease caused by a birnavirus infection is infectious pancreatic necrosis in which case the component comprising the projection domain of a birnavirus VP2 wherein said birnavirus VP2 projection domain lacks the AA' hairpin region derives from IPNV.

[24] A method for obtaining an immunogenic antigen presenting cell specific for a given antigenic entity comprising the steps of:
(i) contacting an immature antigen presenting cell with a conjugate as defined in any of [5] to [16], a polynucleotide as defined in [17] or a vector as defined in [18] under conditions adequate for stimulation of the antigen presenting cell and
(ii) recovering the immunogenic antigen presenting cell.

[25] A method according to [24] wherein the antigen presenting cell is a dendritic cell.

[26] An antigen presenting cell obtainable using the method according to [24] or [25].

[27] A vaccine composition comprising an immunologically active amount of an antigen presenting cell as defined in [26].

[28] An antigen presenting cell as defined in [26] for use in medicine.

[29] An antigen presenting cell as defined in [26] for use in a method of treating a disease caused by a birnavirus infection.

[30] An antigen presenting for use according to [29] wherein
(i) the disease caused by a birnavirus infection is infectious bursal disease in which case the component comprising the projection domain of a birnavirus VP2 and lacking the AA' hairpin region derives from IBDV or
(ii) the disease caused by a birnavirus infection is infectious pancreatic necrosis in which case the component comprising the projection domain of a birnavirus VP2 wherein said birnavirus VP2 projection domain lacks the AA' hairpin region derives from IPNV.

The invention is described herein by way of the following examples which are to be construed as merely illustrative and not limitative of the scope of the invention.

### EXAMPLES

### EXAMPLE 1

### Expression and purification of P HT mAV.

The P_HT_mAV was cloned in a vector suitable for expression in insect cells as an extracellular protein. Briefly, a recombinant baculovirus with the sequences of interest was generated, and it was used to infect insect cells. The recombinant protein, which is secreted to the culture medium, was purified using a cobalt affinity resin. The result of the purification is shown in figure 2.

### EXAMPLE 2

### Coupling P HT mAV and biotinilated CpG oligonucleotide.

To check if the mAV part of the conjugate binds a biotinylated CpG oligonucleotide 1 µg of the purified protein was combined with different amounts of a biotinylated CpG oligonucleotide. The CpG oligonucleótide used was Biotin ODN 2006 (InvivoGen, San Diego, CA-USA). It's sequence is : 5'-TCGTCGTTTTGTCGTTTTGTCGTT-3' (SEQ ID NO: 8) labeled with biotin at the 3' terminus (Bases are phosphorothioate).

P_HT_mAV was incubated 45 minutes at room temperature with CpG at a molecular ratio of 1:0 (without CpG), 1:1, 1:2 and 1:4. Binding was checked by changes in the mobility of the protein in native 0.7% agarose gel electrophoresis followed by western-blotting (Figure 3). A change in the mobility of the protein could be detected even at a 1:1 molecular ratio, indicating that coupling was occurring.

### EXAMPLE 3

### Immunization of chickens with P_HT_mAV and challenge infection

Immunization of chickens is carried out essentially as described by Martinez-Torrecuadrada JL. et al. (Vaccine, 2003, 21:3342-3350).

The required amount of antigen is emulsified with oil in 0.5 ml for each vaccinal dose. A commercial inactivated vaccine, HipraGumboro-BPL2, which contained whole inactivated IBDV virus is used as a standard vaccine.

4-week-old SPF chicks are divided in groups of 20 birds. Birds are inoculated by subcutaneous route in the back of the neck with one dose of the vaccine to be tested. Three groups are inoculated with 5 µg of P_HT_mAV, or 20 µg of P_HT_mAV, or 5 µg of P_HT_mAV:CpG. Commercial vaccine is orally administered to another group of 20 birds. Other group is non-vaccinated and challenged as a challenge control, and other group is kept as a non-vaccinated non-challenge control. At 4 weeks post-vaccination, the animals are blood sampled from the wing vein and sera from birds of the same group are pooled and assayed by serum neutralisation.

After blood sampling, 8-week-old birds are inoculated by oral route with 0.2 ml of a 1/20 dilution in PBS of a bursal homogenate of vvIBDV strain VG-248. The typical IBD symptoms are recorded: ruffled feathers, depression, head between the shoulders and death. At 10 days post-challenge, the surviving animals are sacrificed and necropsied. The bursa of Fabricius (BF)/body weight (BW) ratio is calculated using the formula: (BF weight (in g)/BW (in g)) x 1000. A BF/BW ratio below 2 is considered as a marker of bursal atrophy, which means that the challenge virus has reached the bursa. A portion of the bursa is kept frozen at -80°C and tested later for the presence of IBDV by RT-PCR.

### EXAMPLE 4

### Expression of FlagA HT P, IgY Fc υ3-4 HT P and IgY Fc υ2-4 HT P

As in the case of P_HT_mAV, the sequences encoding the said conjugates have been cloned in a vector suitable for expression in insect cells as an extracellular protein. The expression of the proteins is shown in figure 4.

## Claims

1. A polypeptide comprising a birnavirus VP2 P domain wherein said birnavirus VP2 P domain lacks the AA' hairpin region.

2. A polypeptide as defined in claim 1 wherein the birnavirus is IBDV.

3. A conjugate or kit-of-parts comprising
(i) a first component comprising the projection domain of a birnavirus VP2 wherein said birnavirus VP2 projection domain lacks the AA' hairpin region and
(ii) a second component which is an immune co-stimulatory agent wherein said component (ii) is not a region of said birnavirus VP2.

4. A conjugate as defined in claim 3 wherein said components (i) and (ii) are connected via the interaction between a first member of a binding pair coupled to component (i) and a second member of a binding pair coupled to component (ii).

5. A conjugate as defined in claim 4 wherein the first member of the binding pair is avidin or a functionally equivalent variant thereof and the second member of the binding pair is biotin or wherein the first member of the binding pair is biotin and the second member of the binding pair is avidin or a functionally equivalent variant thereof.

6. A conjugate as defined in claim 3 wherein components (i) and (ii) form a fusion protein.

7. A conjugate as defined in any of claims 3 to 6 wherein the immune co-stimulatory agent is a molecule capable of binding to an antigen-presenting cell selected from the group consisting of a TLR agonist and a Ig Fc region.

8. A conjugate as defined in claim 7 wherein the TLR agonist is selected from the group of consisting of a flagellin and a CpG oligonucleotide.

9. A conjugate as defined in claim 7 wherein the Ig Fc region is the IgY Fc region.

10. A polypeptide as defined in claim 1 or a conjugate as defined in any of claims 3 to 9 wherein the birnavirus is IBDV or IPNV.

11. A polynucleotide encoding a polypeptide as defined in any of claims 1 or 2 or 6 to 10.

12. A host cell comprising a polypeptide as defined in any of claims 1 or 2, a conjugate according to any of claims 3 to 9 or a polynucleotide as defined in claim 11.

13. A vaccine composition comprising an immunologically active amount of a polypeptide as defined in claims 1 or 2, a conjugate according to any of claims 3 to 9 or a polynucleotide as defined in claim 11.

14. A polypeptide as defined in claims 1 or 2, a conjugate according to any of claims 3 to 9 or a polynucleotide as defined in claim 11 for use in a method of treating a disease caused by a birnavirus infection.

15. A polypeptide, conjugate or polynucleotide for use according to claim 14 wherein
(i) the disease caused by a birnavirus infection is infectious bursal disease in which case the component comprising the projection domain of a birnavirus VP2 and lacking the AA' hairpin region derives from IBDV or
(ii) the disease caused by a birnavirus infection is infectious pancreatic necrosis in which case the component comprising the projection domain of a birnavirus VP2 wherein said birnavirus VP2 projection domain lacks the AA' hairpin region derives from IPNV.
